(19) Europäisches Patentamt
European Patent Office
Office européen des brevets

(11) Publication number: 0 383 281 B1

(12) # EUROPEAN PATENT SPECIFICATION

(45) Date of publication of patent specification: 17.08.94

(21) Application number: 90102829.0

(22) Date of filing: 13.02.90

(51) Int. Cl.5: C07C 217/10, C07D 233/64, C07D 333/52, C07D 401/10, C07D 307/78, C07D 295/088, C07D 211/76, C07D 233/58, C07D 211/22, C07D 213/30, C07D 333/16

(54) **1,2-ethanediol derivative and salt thereof, process for producing the same, and cerebral function-improving agent comprising the same.**

(30) Priority: 14.02.89 JP 32714/89
20.03.89 JP 68958/89
26.04.89 JP 106187/89
05.02.90 JP 24501/90
05.02.90 JP 24502/90
05.02.90 JP 24503/90

(43) Date of publication of application: 22.08.90 Bulletin 90/34

(45) Publication of the grant of the patent: 17.08.94 Bulletin 94/33

(84) Designated Contracting States: AT CH DE DK GB LI NL SE

(56) References cited:
WO-A-88/08424
US-A- 2 928 845

Brown, H.C. et Al., J. Am. Chem. Soc., Vol. 110 (1988), pages 1539-1546.

(73) Proprietor: TOYAMA CHEMICAL CO., LTD.
2-5, 3-chome, Nishishinjuku,
Shinjuku-ku
Tokyo 160 (JP)

(72) Inventor: Ono, Satoshi
2-5, Nakajima-3-chome
Toyoama-shi (JP)
Inventor: Yamafuji, Tetsuo
1-3, Yoshitani,
Fuchumachi
Nei-gun, Toyama-ken (JP)
Inventor: Chaki, Hisaaki
455-1, Oharaya,
Oyamamachi
Kaminiikawa-gun, Toyama-ken (JP)
Inventor: Maekawa, Mutsuko
65-5 Shimokumano
Toyama-shi (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 383 281 B1

**CHEMICAL ABSTRACTS, VOL: 63, no. 1, July 5, 1965, Columbus, Ohio, US; B. MACCHIA: "Stereochemistry and orientation of the addition of aminoalcohols to oxides of cis- and transpropenylbenzene", column 557, abstract no. 557**

**CHEMICAL ABSTRACTS, vol. 59, no. 5, September 2, 1963, Columbus, Ohio, US; K. WINTERFELD et al.: "Pehnylsubstitued oxaquinolizidine", column 5157, abstract-no. 5157c**

**JOURNAL OF PHARMACEUTICAL SCIENCES, vol. 50, 1961; S.I. SHAPIRO et al.: "Local anesthetics III. Dialkylamino-alkoxyphenylethanol esters", pages 769-771**

**IL FARMACIO EDIZIONE SCIENTIFICA, VOL: 19, 1964; F. BOTTARI et al.: "E teri del firniglicol e di idrobenzoine con amminoalcoli", pages 1056-1065**

Inventor: **Todo, Yozo**
**2091-12, Ishisaka**
**Toyama-shi (JP)**
Inventor: **Narita, Hirokazu**
**6-40 Okudahonmachi**
**Toyama-shi (JP)**

(74) Representative: **Wächtershäuser, Günter, Prof. Dr.**
**Patentanwalt**
**Tal 29**
**D-80331 München (DE)**

## Description

This invention relates to a 1,2-ethanediol derivative and a salt thereof, a process for producing the same, and a cerebral function-improving agent comprising the same. The cerebral function-improving agent of this invention is useful for treating cerebrovascular dementia, senile dementia, Alzheimer's dementia, sequelae of ischemic encephalopathy and cerebral apoplexy.

As 1,2-ethanediol derivatives, there are known, for example, those described in U.S. Patent No. 2,928,845; J. Pharm. Sci., vol. 50, pp. 769-771 (1961); Farmaco. Ed. Sci., vol. 19, pp. 1056-1065 (1964); etc.

These compounds are in use as a local anesthetic. However, nothing is known as to their use as a cerebral function-improving agent, an antiamnesic agent or a nootropic agent.

WO No. 88/8424 describes that 1,2-ethanediol derivatives can be used for the remedy of Alzheimer's disease and other degenerative neurological disorders. However, no specific description or example of these derivatives is given in the application.

Drugs such as cerebral metabolic enhancers, cerebrovasodilators and the like are currently in use for the remedy of various dementias, particularly dementia of Alzheimer's type and cerebrovascular dementia.

However, no cerebral function-improving agent has been found as yet which is useful for treating cerebrovascular dementia, senile dementia, Alzheimer's dementia, sequelae of ischemic encephalopathy and cerebral apoplexy.

An object of this invention is to provide a novel 1,2-ethanediol derivative and a salt thereof.

Another object of this invention is to provide a process for producing a novel 1,2-ethanediol derivative and a salt thereof.

Still another object of this invention is to provide a novel cerebral function-improving agent which is useful for treating cerebrovascular dementia, senile dementia, Alzheimer's dementia, sequelae of ischemic encephalopathy and cerebral apoplexy and yet has little side effect.

The present inventors have made study in order to solve the above-mentioned problems. As a result, it has been found that a 1,2-ethanediol derivative as defined in claim 1 represented by the following general formula [I] or a salt thereof has an excellent antiamnesic activity and an excellent antihypoxic activity and is very useful as a cerebral function-improving agent:

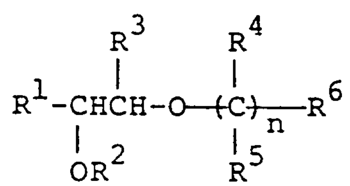

[I]

wherein $R^1$ represents a substituted or unsubstituted heterocyclic group; $R^2$ represents a hydrogen atom, a lower alkyl group or a hydroxyl-protecting group; $R^3$ represents a hydrogen atom or a lower alkyl group; $nR^4$'s and $nR^5$'s are the same as or different from one another and represent hydrogen atoms or lower alkyl groups; $R^6$ represents an ammonio group or a substituted or unsubstituted amino or nitrogen-containing heterocyclic group, the nitrogen-containing heterocyclic group being selected from the group consisting of pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and n represents 0 or an integer of 1 to 6; wherein the substituent on $R^1$ is selected from the group consisting of halogen atoms, substituted or unsubstituted amino, lower alkyl, aryl, ar-lower alkyl, lower alkoxy, ar-lower alkoxy, aryloxy, carbamoyloxy, lower alkylthio, lower alkenyl, lower alkenyloxy, ar-lower alkylthio, ar-lower alkylsulfonyl, arylsulfonyl, lower alkylsulfonylamino, arylsulfonylamino and heterocyclic groups and protected amino groups, protected or unprotected hydroxyl groups, nitro group, oxo group and lower alkylenedioxy groups; the substituted lower alkyl, aryl, ar-lower alkyl, lower alkoxy, ar-lower alkoxy, aryloxy, carbamoyloxy, lower alkylthio, lower alkenyl, lower alkenyloxy, ar-lower alkylthio, ar-lower alkylsulfonyl, arylsulfonyl, lower alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected or unprotected hydroxyl groups, protected or unprotected amino groups, protected or unprotected carboxyl groups, unsubstituted lower alkyl groups, lower alkyl groups substituted by a protected or unprotected hydroxyl group, unsubstituted or halogen-substituted aryl groups, unsubstituted or

halogen-substituted aroyl groups, unsubstituted lower alkoxy groups, lower alkoxy groups substituted by a lower alkoxy group, lower acyl groups, ar-lower alkyl groups, ar-lower alkenyl groups, heterocyclic groups, heterocyclic-CO- groups, oxo group, lower alkylsulfonyl groups and arylsulfonyl groups; and the substituted amino group as the substituent of $R^1$ and the substituted amino group as $R^6$ have each at least one substituent selected from the group consisting of protected or unprotected hydroxyl groups, unsubstituted lower alkyl groups, lower alkyl groups substituted by a protected or unprotected carboxyl or hydroxyl group, cycloalkyl groups, aryl groups, lower acyl groups, ar-lower alkyl groups, heterocyclic groups, unsubstituted or oxo-substituted heterocyclic-CO- groups, adamantyl group, lower alkylsulfonyl groups and arylsulfonyl groups; all the above heterocyclic groups being selected from the group consisting of the nitrogen-containing heterocyclic groups mentioned in the definition of $R^6$ and furyl, thienyl, benzothienyl, pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo-[2,3-a]pyridyl, benzo[b]piperazinyl, chromenyl, isothiazolyl, isoxazolyl, oxadiazolyl, pyridazinyl, isoindolyl and isoquinolyl groups.

Incidentally, the cerebral function-improving agent mentioned herein refers to a cerebral function-improving agent having not only effects possessed by conventional cerebral function-improving agents, for example, sequelae of ischemic encephalopathy and cerebral apoplexy but also therapeutic or prophylactic effects for amnesia and dementias (e.g. cerebrovascular dementia, senile dementia and Alzheimer's dementia).

In the present specification, the following terms have the following definitions unless otherwise specified.

The term "halogen atom" means, for example, a fluorine atom, a chlorine atom, a bromine atom and an iodine atom; the term "lower alkyl group" means $C_{1-6}$ alkyl groups such as methyl, ethyl, n-propyl, isopropyl, n-butyl, isobutyl, tert-butyl, pentyl, hexyl and the like; the term "lower alkoxy group" means $C_{1-6}$ alkyl-O- groups; the term "lower alkylthio group" means $C_{1-6}$ alkyl-S-groups; the term "lower alkenyl group" means $C_{2-6}$ alkenyl groups such as vinyl, propenyl, butenyl, pentenyl, hexenyl and the like; the term "lower alkenyloxy group" means $C_{2-6}$alkenyl-O- groups; the term "cycloalkyl group" means $C_{3-6}$cycloalkyl groups such as cyclopropyl, cyclobutyl, cyclopentyl, cyclohexyl and the like; the term "aryl group" means phenyl, naphthyl, indanyl and indenyl groups; the term "aryloxy group" means aryl-O-groups; the term "ar-lower alkyl group" means ar-$C_{1-4}$ alkyl groups such as benzyl, diphenylmethyl, trityl, phenethyl and the like; the term "ar-lower alkoxy group" means ar-$C_{1-4}$alkyl-O- groups; the term "ar-lower alkylthio group" means aryl-$C_{1-4}$alkyl-S- groups; the term "lower alkylenedioxy group" means $C_{1-4}$alkylenedioxy groups such as methylenedioxy, ethylenedioxy and the like; the term "lower acyl group" means $C_{1-6}$acyl groups such as formyl, acetyl, butyryl and the like; the term "aroyl group" means aryl-CO-groups; the term "lower alkylsulfonyl group" means $C_{1-6}$alkyl-$SO_2$- groups; the term "arylsulfonyl group" means aryl-$SO_2$- groups; ther term "ar-lower alkylsulfonyl group" means aryl-$C_{1-6}$alkyl-$SO_2$- groups; the term "lower alkylsulfonyloxy group" means $C_{1-6}$alkyl-$SO_2$-O- groups; the term "aryl-sulfonyloxy group" means aryl-$SO_2$-O- groups; the term "lower alkylsulfonylamino group" means $C_{1-6}$alkyl-$SO_2$-NH-groups; the term "arylsulfonylamino group" means aryl-$SO_2$NH- groups; the term "di-lower alkylamino group" means di-$C_{1-6}$alkyl-NH- groups and the term "ammonio group" means tri-lower alkylammonio groups such as trimethylammonio, triethylammonio; the term "lower alkoxycarbonyl group" means $C_{1-6}$alkyl-O-CO-groups and the like.

The protective groups for hydroxyl group, carboxyl group and amino group include those conventional protective groups for hydroxyl group, carboxyl group and amino group which are described in Protective Groups in Organic Synthesis [Theodra W. Greene (1981) John Wiley & Sons, Inc.]. In particular, the protective group for hydroxyl group specifically includes, for example, lower alkyl, lower acyl; tetrahydropyranyl and ar-lower alkyl groups such as substituted or unsubstituted benzyl.

The salt of the 1,2-ethanediol derivative represented by the general formula [I] can be any pharmaceutically acceptable salt. It includes, for example, salts with mineral acids such as hydrochloric acid, hydrobromic acid, sulfuric acid, phosphoric acid and the like; salts with carboxylic acids such as formic acid, acetic acid, oxalic acid, fumaric acid, maleic acid, malic acid, tartaric acid, aspartic acid and the like; salts with sulfonic acids such as methanesulfonic acid, benzenesulfonic acid, p-toluenesulfonic acid, naphthalenesulfonic acid and the like; and salts with alkali metals such as sodium, potassium and the like.

When the 1,2-ethanediol derivative of the general formula [I] or its salt has isomers (e.g. optical isomers, geometrical isomers, tautomers), all of these isomers are included in this invention. Also, the hydrate, solvate and all crystal forms of the compound of this invention are included in this invention.

Next, there is described a process for producing a 1,2-ethanediol derivative of the general formula [I] or a salt thereof.

The 1,2-ethanediol derivative of the general formula [I] or its salt can be produced by per se known processes or their appropriate combinations, for example, the following production processes.

## Production Process 1

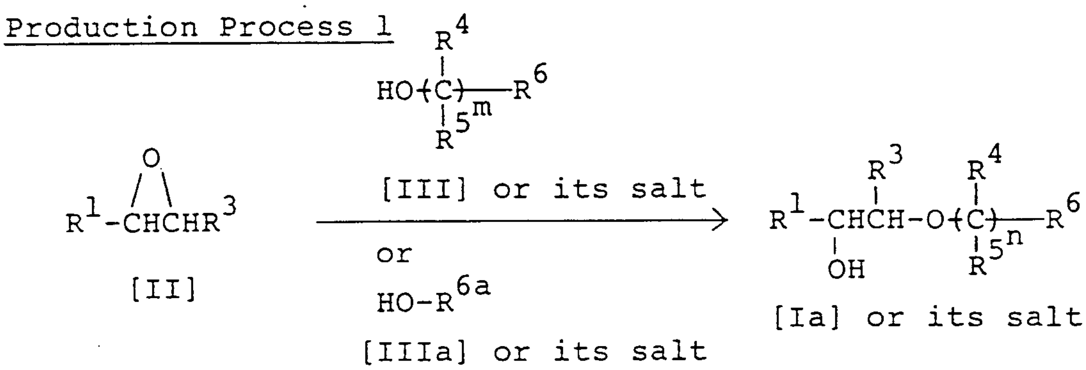


## Production Process 2

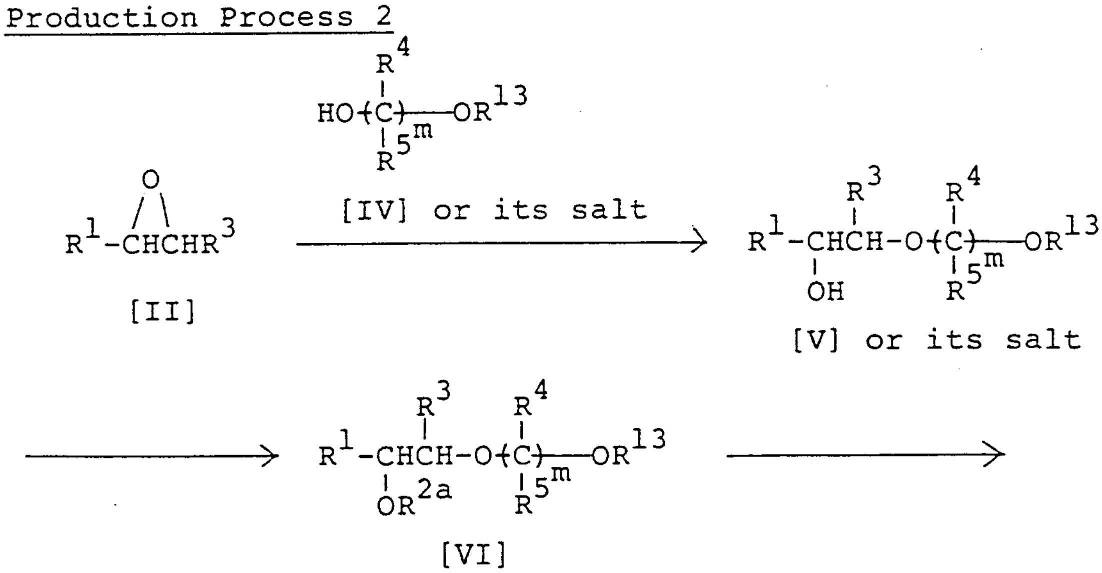

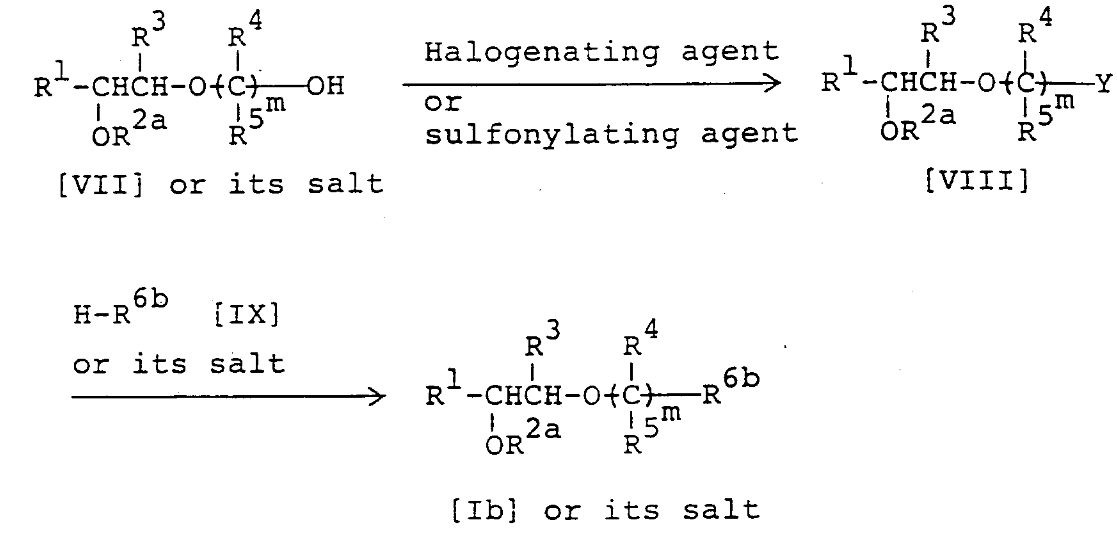


<u>Production Process 3</u>

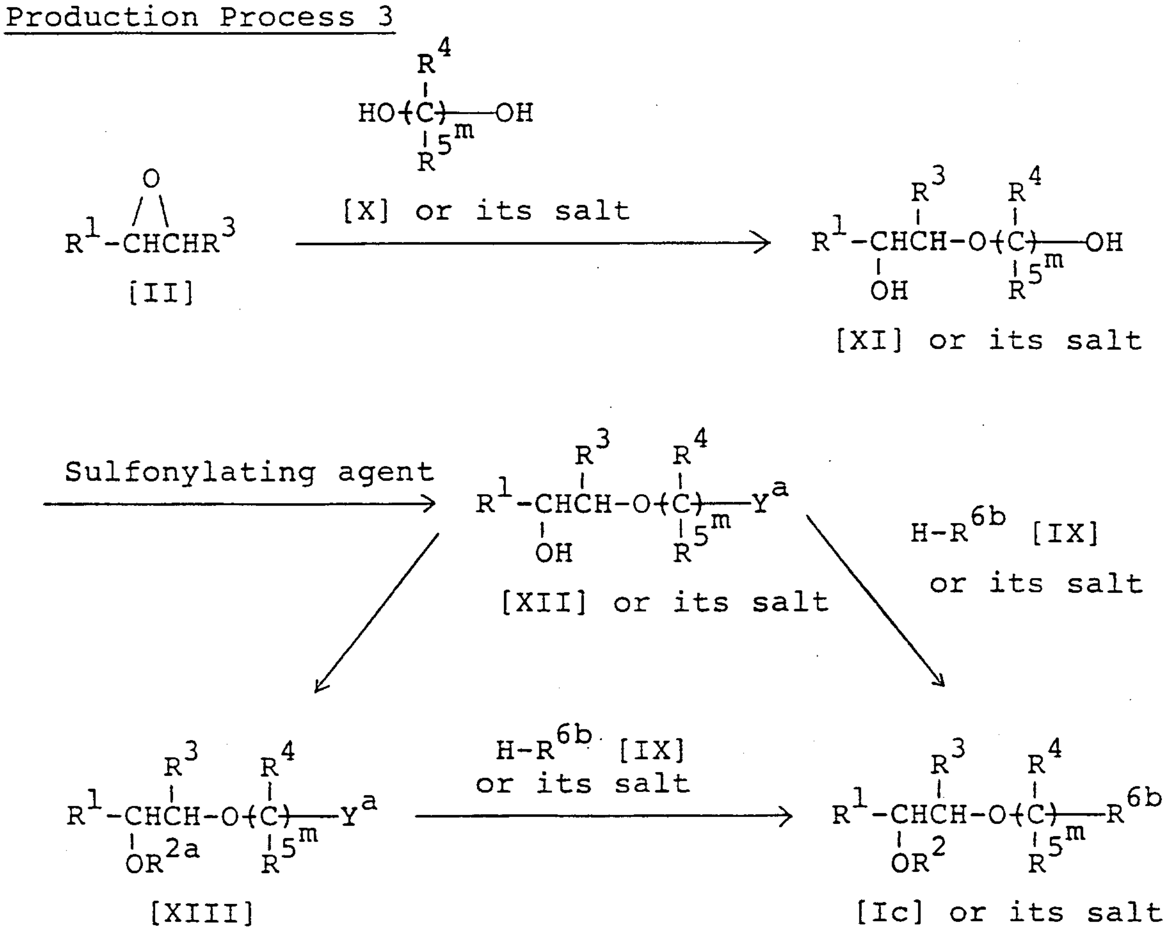

Production Process 4

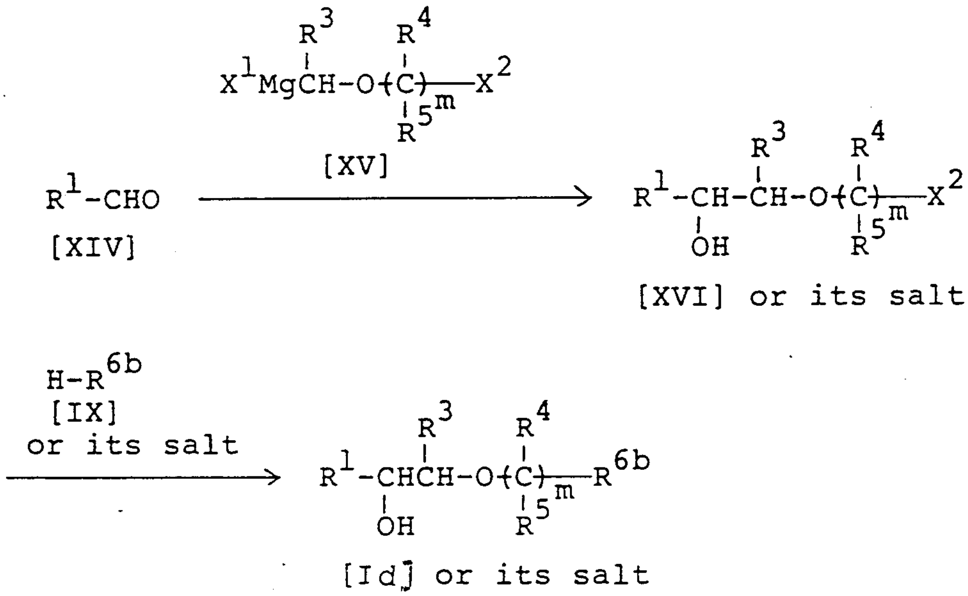

[XIV]

[XV]

[XVI] or its salt

[IX]

or its salt

[Id] or its salt

In the above reaction schemes, $R^1$, $R^2$, $R^3$, $R^4$, $R^5$, $R^6$ and n have the same meanings as defined above; $R^{2a}$ represents the same hydroxyl-protecting group as in the definition of $R^2$; $R^{6a}$ represents the same substituted or unsubstituted nitrogen-containing heterocyclic group as in the definition of $R^6$, provided that the heterocyclic group has a free valence on a carbon atom forming the heterocyclic ring; $R^{6b}$ represents the same substituted or unsubstituted nitrogen-containing heterocyclic group as in the definition of $R^6$, provided that the nitrogen-containing heterocyclic group has a free valence on a nitrogen atom forming the heterocyclic ring, or a substituted or unsubstituted amino group; $R^{13}$ represents the same hydroxyl-protecting group as in the definition of $R^2$; $X^1$ and $X^2$, which may be the same or different, represent halogen atoms, Y represents a removable group such as a halogen atom, a lower alkylsulfonyloxy group, an arylsulfonyloxy group or the like; $Y^a$ represents an arylsulfonyloxy group; and m represents an integer of 1-6.

As the salts of the compounds of the general formulas [III], [IIIa], [IV], [V], [VII], [IX], [X], [XI], [XII], [XVI], [Ia], [Ib], [Ic] and [Id], there can be mentioned the same salts as the salts of the compound of the general formula [I].

Next, each of the above production processes is described.

Production Process 1

A compound of the general formula [II] is reacted with a compound of the general formula [III] or its salt or a compound of the general formula [IIIa] or its salt in the presence or absence of a base to obtain a compound of the general formula [Ia] or its salt.

The solvent to be used in this reaction can be any solvent unless it adversely affects the reaction. There can be mentioned, for example, aromatic hydrocarbons such as benzene, toluene, xylene and the like; sulfoxides such as dimethylsulfoxide and the like; amides such as N,N-dimethylformamide and the like; and ethers such as tetrahydrofuran, dioxane and the like. These solvents can be used alone or in admixture of two or more. It is also possible to use the compound of the general formula [III] or [IIIa] as the solvent.

As the base to be used optionally, there can be mentioned, for example, sodium hydride, metallic sodium and potassium tert-butoxide.

In the reaction, the compound of the general formula [III] or its salt, or the compound of the general formula [IIIa] or its salt is used in an amount of 1-100 moles, preferably 1-10 moles, per mole of the compound of the general formula [II].

The base as an optional component is used in an amount of 0.01-1.2 moles per mole of the compound of the general formula [II].

This reaction can be effected usually at 20-150 °C, preferably 70-90 °C, for 1 minutes to 24 hours, preferably 5 minutes to 5 hours.

Production Process 2

(1) A compound of the general formula [II] is reacted with a compound of the general formula [IV] or its salt in the presence or absence of a base to obtain a compound of the general formula [V] or its salt.

This reaction can be effected in the same manner as described in Production Process 1.

The obtained compound of the general formula [V] or its salt may be used in the subsequent reaction without being isolated.

(2) The compound of the general formula [V] or its salt is subjected to conventional reaction for protection of hydroxyl group to obtain a compound of the general formula [VI].

The obtained compound of the general formula [VI] may be used in the subsequent reaction without being isolated.

Then, the compound of the general formula [VI] is subjected to reaction for selective removal of the hydroxyl-protecting group to obtain a compound of the general formula [VII] or its salt.

The obtained compound of the general formula [VII] or its salt may be used in the subsequent reaction without being isolated.

These reactions can be effected according to per se known methods, for example, the method described in Protective Groups in Organic Synthesis [Theodra W. Green (1981), John Wiley & Sons, Inc.] or a method similar thereto.

The combination of the hydroxyl-protecting groups ($R^{13}$ and $R^{2a}$) to be used in these reactions can be selected appropriately.

(3) The compound of the general formula [VII] or its salt is reacted with a halogenating agent or a sulfonylating agent in a solvent in the presence or absence of a base to obtain a compound of the general formula [VIII].

The solvent to be used in the reaction can be any solvent unless it adversely affects the reaction. There can be mentioned, for example, halogenated hydrocarbons such as methylene chloride, chloroform and the like; ethers such as tetrahydrofuran, dioxane and the like; nitriles such as acetonitrile and the like; and amides such as N,N-dimethylformamide and the like. These solvents can be used alone or in admixture of two or more.

As the base to be used optionally, there can be mentioned, for example, organic and inorganic bases such as triethylamine, diisopropylethylamine, 1,8-diazabicyclo-[5.4.0]undec-7-ene (DBU), pyridine, potassium tert-butoxide, sodium carbonate, potassium carbonate, sodium hydride and the like.

As the halogenating agent, there can be mentioned, for example, phosphorus oxychloride, phosphorus oxybromide, phosphorus trichloride, phosphorus pentachloride, thionyl chloride and the like.

As the sulfonylating agent, there can be mentioned, for example, methanesulfonyl chloride, p-toluenesulfonyl chloride and the like.

Each of the halogenating agent, the sulfonylating agent and the base as an optional component is used in an amount of at least 1 mole, preferably 1-2 moles, per mole of the compound of the general formula [VII] or its salt.

This reaction can be effected usually at -10° to 100°C, preferably 0° to 40°C, for 10 minutes to 30 hours.

The obtained compound of the general formula [VIII] may be used as it is, in the subsequent reaction without being isolated.

(4) The compound of the general formula [VIII] is reacted with a compound of the general formula [IX] or its salt in the presence or absence of a catalyst in the presence or absence of a base to obtain a compound of the general formula [Ib] or its salt.

The solvent to be used in this reaction can be any solvent unless it adversely affects the reaction. There can be mentioned, for example, the same solvents as mentioned in (3) of Production Process 2.

As the catalyst to be used optionally, there can be mentioned, for example, potassium iodide, sodium iodide and the like.

The catalyst is used in an amount of 0.1-1 mole per mole of the compound of the general formula [VIII].

As the base to be used optionally, there can be mentioned, for example, the same bases as mentioned in (3) of Production Process 2.

Each of the compound of the general formula [IX] or its salt and the base as an optional component is used in an amount of at least 1 mole, preferably 1-20 moles, per mole of the compound of the general formula [VIII].

This reaction can be effected usually at 10-150°C, preferably 20-100°C for 10 minutes to 20 hours.

Production Process 3

(1) A compound of the general formula [II] is reacted with a compound of the general formula [X] or its salt in the presence or absence of a base to obtain a compound of the general formula [XI] or its salt.

This reaction can be effected in accordance with the same method as mentioned in Production Process 1.

(2) The compound of the general formula [XI] or its salt is reacted with a sulfonylating agent in a solvent in the presence or absence of a base to obtain a compound of the general formula [XII] or its salt.

The solvent to be used in this reaction can be any solvent unless it adversely affects the reaction. There can be mentioned, for example, the same solvents as mentioned in (3) of Production Process 2.

As the base to be used optionally, there can be mentioned, for example, the same bases as mentioned in (3) of Production Process 2.

As the sulfonylating agent, there can be mentioned, for example, p-toluenesulfonyl chloride and the like.

Each of the sulfonylating agent and the base as an optional component is used in an amount of at least 0.95 mole, preferably 1-2 moles, per mole of the compound of the general formula [XI] or its salt.

This reaction can be effected usually at -10° to 100°C, preferably 0° to 40°C, for 10 minutes to 30 hours.

The obtained compound of the general formula [XII] or its salt may be used in the subsequent reaction without being isolated.

(3) The compound of the general formula [XII] or its salt is subjected to conventional reaction for protection of hydroxyl group to obtain a compound of the general formula [XIII].

The reaction can be effected in accordance with per se known methods, for example, the method described in Protective Groups in Organic Synthesis [Theodra W. Green (1981), John Wiley & Sons, Inc.] or a method similar thereto.

The obtained compound of the general formula [XIII] may be used in the subsequent reaction without being isolated.

(4) The compound of the general formula [XII] or its salt or the compound of the general formula [XIII] is reacted with a compound of the general formula [IX] or its salt in the presence or absence of a base to obtain a compound of the general formula [Ic] or its salt.

This reaction can be effected in the same method as described in (4) of Production Process 2.

Production Process 4

(1) A compound of the general formula [XIV] is reacted with a compound of the general formula [XV] to obtain a compound of the general formula [XVI] or its salt.

The solvent to be used in this reaction can be any solvent unless it adversely affects the reaction. There can be mentioned, for example, ethers such as diethyl ether, tetrahydrofuran, dioxane and the like; and aromatic hydrocarbons such as benzene, toluene and the like. These solvents can be used alone or in admixture of two or more.

In this reaction, the compound of the general formula [XV] is used in an amount of 0.8-100 moles, preferably 0.8-10 moles, per mole of the compound of the general formula [XIV].

This reaction can be effected usually at -78° to 100°C, preferably -78° to 50°C for 5 minutes to 24 hours.

The obtained compound of the general formula [XVI] or its salt may be used in the subsequent reaction without being isolated.

Incidentally, the compound of the general formula [XV] to be used in this reaction can be produced according to a per se known method, for example, the method described in Bull. Soc. Chim. Fr., 1967 (5), pp. 1533-40.

(2) The compound of the general formula [XVI] or its salt is reacted with a compound of the general formula [IX] or its salt in the presence or absence of a base to obtain a compound of the general formula [Id] or its salt.

The solvent to be used in this reaction can be any solvent unless it adversely affects the reaction. There can be mentioned, for example, halogenated hydrocarbons such as methylene chloride, chloroform and the like; ethers such as tetrahydrofuran, dioxane and the like; alcohols such as ethanol, propanol, butanol and the like; nitriles such as acetonitrile and the like; and amides such as N,N-dimethylformamide and the like. These solvents can be used alone or in admixture of two or more.

As the base to be used optionally, there can be mentioned, for example, the same bases as mentioned in (3) of Production Process 2.

Each of the compound of the general formula [IX] or its salt and the base as an optional component is used in an amount of at least 1 mole, preferably 1-20 moles, per mole of the compound of the general formula [XVI] or its salt.

This reaction can be effected usually at 10-150 °C, preferably 20-100 °C, for 10 minutes to 20 hours.

In the above production processes, the reactants or base can also be used as solvent depending on the nature of the reactants or base.

In the above production processes, when the compounds of the general formulas [II], [III], [IIIa], [IV], [V], [VI], [VII], [VIII], [IX], [X], [XI], [XII], [XIII], [XIV], [XV] and [XVI], have isomers (e.g. optical isomers, geometrical isomers, tautomers), the compounds can be used in any isomer form. Further, the compounds can be used in a hydrate form, a solvate form or any crystal form.

When the compounds of the general formulas [II], [III], [IIIa], [IV], [V], [VI], [VII], [VIII], [IX], [XII], [XIII], [XIV], [XV], [XVI], [I], [Ia], [Ib], [Ic] and [Id] have a hydroxyl group, an amino group or a carboxyl group, it is possible to previously protect these groups with a conventional protective group and to remove, after the reaction, the protective group, if necessary, according to a per se known method.

The compound thus obtained may be subjected to conventional isolation and purification procedures such as column chromatography, crystallization, distillation, extraction and the like.

The 1,2-ethanediol derivative of the general formula [I] or its salt can be converted into other 1,2-ethanediol derivative of the general formula [I] or its salt by subjecting the former compound to appropriate combination of per se known reactions such as oxidation reaction, reduction reaction, addition reaction, acylation reaction, alkylation reaction, sulfonylation reaction, deacylation reaction, substitution reaction, dehydration reaction, hydrolysis reaction and the like.

The compound of the general formula [II] which is the starting material for producing the compound of this invention can be produced by per se known processes, for example, the process described in JACS, vol. 87, p.1353 (1965) and the process described in Shin Jikken Kagaku Koza, vol. 14, p. 579 (1977), Maruzen.

The compound of this invention, when used as a drug, may be appropriately mixed with excipients such as filler, carrier, diluent and the like and can be formed into tablets, capsules, powders, granules, fine granules, pills, suspensions, emulsions, liquids, syrups, injections, etc. according to conventional methods. These drugs can be administered orally or parenterally. The dosage route, dose and number of administrations can be appropriately varied depending upon the age, weight and symptom of patient, but in the case of oral administration, generally 0.01-500 mg of the present compound can be administered daily to an adult patient in one to several portions.

Next, the pharmacological activities of representative compounds of this invention are described.

The numbers of the test compounds used in the following pharmacological tests refer to the numbers of the compounds shown in Production Examples appearing hereinafter.

1. Effect of test compound on hypoxia

A test compound dissolved in physiological saline (100 mg/kg) was orally administered to a ddY female mouse (5-6 weeks old, each group consisting of 10 mice). After 1 (or 30 minutes*) hour from the administration, each mouse was placed in a 300-ml glass chamber, and a gas mixture consisting of 4% of oxygen and 96% of nitrogen was passed through the chamber at a rate of 5 liters/min. A time from the start of gas passing to the death of each mouse was measured.

To a control mice group was orally administered only physiological saline.

The antihypoxic activity of the test compound was calculated from the following formula:

$$\frac{\text{Mean survival time of mice of administration group}}{\text{Mean survival time of mice of control group}} \times 100\ (\%)$$

The results are shown in Table 1.

Table 1

| Compound No. | Antihypoxic Activity (%) | Compound No. | Antihypoxic Activity (%) |
|---|---|---|---|
| 1 | 221* | 31 | 157* |
| 2 | 154* | 32 | 160* |
| 9 | 212* | 33 | 268* |
| 14 | 217* | 34 | 185* |
| 16 | 160* | 36 | 162* |
| 19 | 242* | 37 | 251* |
| 21 | 230* | 38 | 209* |
| 23 | 246* | 39 | 147* |
| 25 | 224* | 42 | 132* |
| 26 | 309* | 46 | 189* |
| 27 | 144* | 47 | 211* |
| 28 | 131* | 54 | 155* |
| 29 | 163* | 57 | 135* |
| | | 58 | 143* |
| | | 64 | 156* |
| | | 66 | 221* |
| | | 72 | 293* |
| | | 74 | 161* |
| | | 75 | 169* |
| | | 76 | 228* |
| | | 77 | 213* |
| | | 79 | 248* |
| | | 83 | 241* |
| | | 85 | 316* |
| | | 87 | 171* |
| | | 94 | 137* |

Note: * Mice were placed in the chamber after 30 minutes from the administration instead of after 1 hr from the administration.

2. Effect of test compound on amnesia

(1) Electroconvulsive shock (ECS)-induced amnesia

A test compound dissolved in physiological saline was intraperitoneally administered to a ddY male mouse (5-6 weeks old, each group consisting of 10 mice). The acquisition trial in passive avoidance task was carried out after 1 hour from the administration. Each mouse was placed in the bright compartment of a two-compartment step-through type passive avoidance apparatus consisting of a bright compartment and a dark compartment (MPA-100M manufactured by Muromachi Kikai). When the mouse entered the dark compartment, the guilotine door of the dark compartment was closed; after 0.5 second, an inescapable footshock (1.6 mA, 3 seconds) was delivered. Immediately thereafter, ECS (25 mA, 0.5 second) was applied through the both eyes of the mouse. After 24 hours, in the retention trial, the mouse was again placed in the bright compartment and the response latency for mouse to enter the dark compartment was measured. If the mouse avoided longer than 300 seconds, a ceiling score of 300 seconds was assigned.

A control mice group to which only physiological saline had been administered intraperitoneally, and response latency was also measured in the same manner.

Antiamnesic activity was taken as a median of the response latencies of the 10 mice and expressed by the following symbols:

- : 0-60 seconds
+ : 61-100 seconds
+ + : 101-150 seconds
+ + + : 151-300 seconds

The results are shown in Table 2.

Table 2

| Compound No. | Dosage (mg/Kg) | Antiamnesic activity |
|---|---|---|
| 4 | 3 | + + |
| 5 | 3 | + |
| 8 | 10 | + + |

(2) Effect of test compound on cycloheximide-induced amnesia

It was reported by Yamazaki et al. [Drugs, Mind and Action, vol. 3, pp. 127-136 (1983)] that cycloheximide interfers with the retrieval process of memory of mouse. Hence, the following test was carried out.

A test was carried out in accordance with the method described in Drugs, Mind and Action, vol. 3, pp. 127-136 (1983) and Folia Pharmacologica Japonica, vol. 89, pp. 243-252 (1987).

As the test apparatus, there was used a step-down type passive avoidance training box. It was a black acrylic resin box of 22 cm x 22 cm x 21 cm (height) whose floor portion consisted of a stainless steel grid and which had, at one corner of the floor grid, a platform of 7 cm x 7 cm x 2 cm (height).

Cycloheximide was dissolved in physiological saline, and injected subcutaneously at 120 mg/kg to a ddY male mouse (5-6 weeks old, each group consisting of 10 mice). In an acquisition trial, after 15 minutes from the administration, each mouse was placed on the platform in the above test apparatus. As soon as the mouse stepped down the platform, a 2 mA current was delivered for 2 seconds, immediately after which the mouse was returned to its home cage. A retention test was performed 24 hours after the acquisition. To each mouse which had been treated with cycloheximide was orally administered a test compound dissolved in physiological saline; after 30 minutes from the administration, the mouse was again placed on the platform and the response latency for mouse to step down was measured. If the mouse avoided longer than 300 seconds, a ceiling score of 300 seconds was assigned.

A control mice group to which only physiological saline had been administered orally, and response latency was also measured in the same manner.

Antiamnesic activity was taken as a median of the response latencies of the 10 mice and expressed by the following symbols:

- : 0-60 seconds

+ : 61-100 seconds
+ + : 101-150 seconds
+ + + : 151-300 seconds

The results are shown in Table 3.

Table 3

| Compound No. | Dosage (mg/Kg) | Antiamnesic activity |
|---|---|---|
| 1 | 10 | + |
| 6 | 3 | + + |
| 9 | 3 | + + |
| 13 | 10 | + + |
| 14 | 10 | + + |
| 22 | 3 | + |
| 23 | 3 | + |
| 27 | 3 | + + |
| 28 | 10 | + |
| 29 | 3 | + |
| 30 | 3 | + |
| 32 | 10 | + + |
| 35 | 3 | + |
| 39 | 3 | + + |
| 40 | 3 | + |
| 41 | 3 | + + |
| 42 | 3 | + |
| 47 | 10 | + + |
| 49 | 10 | + |
| 54 | 3 | + |
| 56 | 3 | + + + |
| 57 | 3 | + |
| Control | - | - |

3. Inhibitory activity for acetylcholinesterase

A test was conducted in accordance with the method of Ellman et al. [Biochem. Pharmacol., vol. 7, pp. 88-95, 1961].

That is, acetylthiocholine (as a substrate) was added to a phosphate buffer solution containing 5,5'-dithiobis-(2-nitrobenzoic acid) (DTNB), a test compound and a mouse cerebral homogenate (as an acetylcholinesterase source). The resulting mixture was incubated, and the amount of the resulting 5-thio-2-nitrobenzoic acid was photometrically measured at 412 nm.

The inhibitory activity for acetylcholinesterase of the test compound was expressed by the inhibition (%) when the final concentration of the test compound was 10 $\mu$g/ml.

The results are shown in Table 4.

Table 4

| Compound No. | Inhibition (%) | Compound No. | Inhibition (%) |
|---|---|---|---|
| 8 | 30 | 30 | 61 |
| 11 | 20 | 31 | 67 |
| 18 | 45 | 39 | 42 |
| 25 | 21 | 41 | 26 |
| | | 61 | 30 |
| | | 66 | 23 |
| | | 75 | 47 |
| | | 94 | 59 |

4. Acute toxicity

A test compound dissolved in physiological saline was intravenously administered to a group of 3 ddY male mice (5-6 weeks old) to examine the acute toxicity of the test compound.

As a result, test compound Nos. 1, 2, 4, 5, 8, 14, 16, 26, 49, 57, 58, 64, 66, 74, 79, and 85 gave no death case at 50 mg/kg.

It is easily appreciated from the above test results that the compound of this invention is excellent in antihypoxic activity, antiamnesic activity and inhibitory activity for acetylcholinesterase and has low toxicity.

From the above result, it is also easily appreciated that the cerebral function-improving agent of this invention is useful for treating cerebrovascular dementia, senile dementia, Alzheimer's dementia, sequelae of ischemic encephalopathy and cerebral apoplexy.

Next, the process for producing the compound of this invention is specifically described by way of Production Examples.

In the Production Examples, the mixing ratio of eluant is by volume in all cases and, as the carrier in column chromatography, there was used a silica gel (Kieselgel 60, Art. 7734) manufactured by Merck Co.

The abbreviations used in the Production Examples have the following meanings.

Me: methyl, Et: ethyl, i-Pr: isopropyl, Ac: acetyl, Ph: phenyl, Bz: benzyl, Tr: trityl, IPA: isopropyl alcohol, IPE: diisopropyl ether.

In the following sentences and tables, the substances in [ ] refer to solvents used in recrystallization.

Production Example 1

1.7 g of potassium tert-butoxide was added to 31 ml of 2-(N,N-dimethylamino)ethanol. The resulting mixture was heated to 80 °C. To the solution was dropwise added, at 80-85 °C in 1.5 hours, a solution of 5.2 g of 2-(benzo[b]thiophen-5-yl)oxirane dissolved in 8 ml of dimethyl sulfoxide. The resulting mixture was stirred at the same temperature for 1 hour. The reaction mixture was cooled and added to a mixture of 60 ml of ethyl acetate and 60 ml of ice water. The organic layer was separated. The aqueous layer was extracted with 30 ml of ethyl acetate. The extract was combined with the previously separated organic layer. To the combined organic layer was added 50 ml of ice water and the resulting mixture was adjusted to pH 1.5 with 6 N hydrochloric acid. The aqueous layer was separated and 50 ml of chloroform was added thereto. The resulting mixture was adjusted to pH 10.5 with potassium carbonate. The organic layer was separated, washed with water and dried over anhydrous magnesium sulfate. The solvent was removed by distillation under reduced pressure. The resulting oily product was dissolved in 50 ml of acetone. To the solution was added 4.3 ml of a 5 N dry hdyrogen chloride-ethanol solution. The resulting mixture was stirred at room temperature for 1 hour and 20 ml of diethyl ether was then added thereto. The resulting mixture was further stirred for 1 hour. The resulting crystals were collected by filtration and dried to obtain 3.3 g of 1-(benzo[b]-thiophen-5-yl)-2-[2-(N,N-dimethylamino)ethoxy]ethanol hydrochloride (compound No. 1).

Melting point: 191.5-192.5 °C [EtOH-$Me_2CO$]

The compounds shown in Table 5 were obtained in the same manner.

In Table 5, $R^1$, $R^2$, $R^3$, $R^4$, $R^6$ and n each show a substituent or integer used in the following formula:

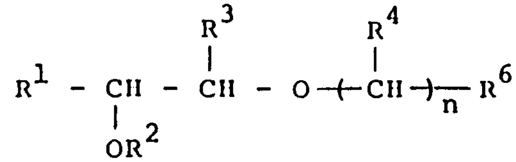

Table 5

| Compound No. | $R^1$ | $R^2$ | $R^3$ | $R^4$ | $R^6$ | n | Addition salt | Melting point (°C) |
|---|---|---|---|---|---|---|---|---|
| 2 | 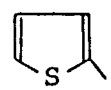 | H | H | H | 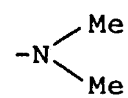 | 2 | HCl | 139-141 |
| 3 | 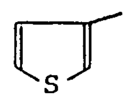 | " | " | " | " | " | " | 165.5-166 [EtOH-$Et_2O$] |
| 4 | 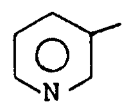 | " | " | " | " | " | 2HCl | 143-146 [EtOH] |
| 5 | 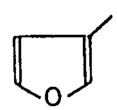 | " | " | " | " | " | HCl | 128.5-130 [EtOH] |

Table 5 (Cont'd)

| | | | | | | | | |
|---|---|---|---|---|---|---|---|---|
| 6 | 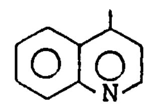 | H | H | H | 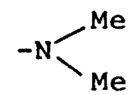 | 2 | 2HCl | 185-185.5 [EtOH-IPA] |
| 7 | 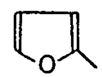 | " | " | " | " | " | HCl | 128-130 |
| 8 | 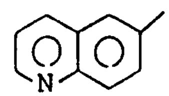 | " | " | " | " | " | Fumaric acid | 136-136.5 [IPA] |
| 9 | 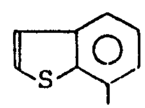 | " | " | " | " | " | HCl | 166.5-167.5 [IPA-AcOEt] |
| 10 | 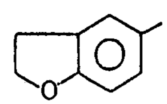 | " | " | " | " | " | " | 168.5-169.5 [IPA] |

Table 5 (Cont'd)

| | | | | | | | | |
|---|---|---|---|---|---|---|---|---|
| 11 | 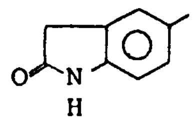 | H | H | H | 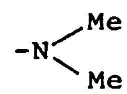 | 2 | HCl | 169-170 |
| 12 | 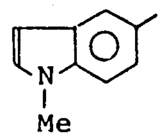 | " | " | " | " | " | - | Oily |
| 13 | 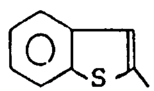 | " | " | " | " | " | " | 188.5-189 [EtOH-AcOEt] |
| 14 | 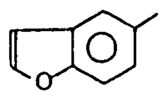 | " | " | " | " | " | " | 168-169.5 [IPA-AcOEt] |
| 15 | 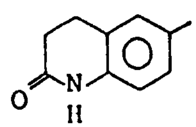 | " | " | " | " | " | " | 169-172 [EtOH] |

Table 5 (Cont'd)

| | | | | | | | | |
|---|---|---|---|---|---|---|---|---|
| 16 | 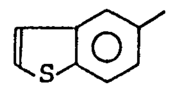 | H | H | H | 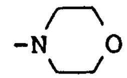 | 2 | HCl | 166.5-167.5 [EtOH-$Me_2CO$] |
| 17 | 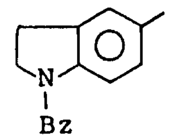 | " | " | " | 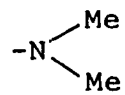 | " | - - | Oily |
| 18 * | 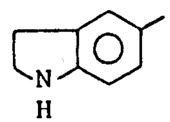 | " | " | " | " | " | 2HCl | " |
| 19 | 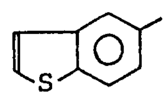 | " | " | " | 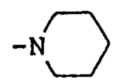 | " | HCl | 167.5-169 |
| 20 | 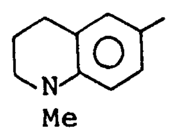 | " | " | " | 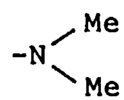 | " | 2HCl | Oily |

Table 5 (Cont'd)

| | | | | | | | | |
|---|---|---|---|---|---|---|---|---|
| 21 | 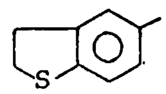 | H | H | H | 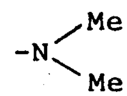 | 2 | HCl | 207.5-210 [EtOH] |
| 22 | 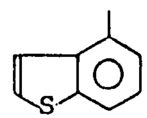 | " | " | " | " | " | " | 190.5-192 [EtOH-IPA] |
| 23 | 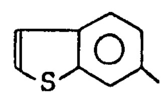 | " | " | " | " | " | " | 171-172 [IPA-AcOEt] |
| 24 | 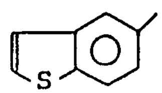 | " | " | " | 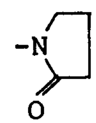 | " | - | Oily |
| 25 | " | " | " | " | 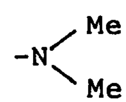 | 3 | - | 81.5-85 |

Table 5 (Cont'd)

| | | | | | | | | |
|---|---|---|---|---|---|---|---|---|
| 26 | 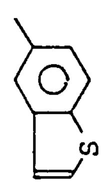 | H | H | H | 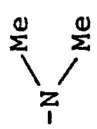 | 4 | HCl | 105-107 [EtOH-AcOEt] |
| 27 | 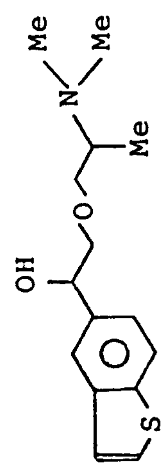 | | | | | | Fumaric acid | 123-124.5 [EtOH-AcOEt] |

Note: *: This compound can be obtained by subjecting compound No. 17 to conventional hydrogenation reaction.

Production Example 2

A mixture of 1.6 g of 3-pyridinemethanol, 1.7 g of potassium tert-butoxide and 23 ml of dimethyl sulfoxide was heated to 80 °C. Thereto was added 2.4 g of 2-(benzo[b]furan-5-yl)oxirane. The resulting

mixture was stirred at 85-90 °C for 15 minutes. The reaction mixture was added to a mixture of 50 ml of ice water and 50 ml of ethyl acetate. The resulting mixture was adjusted to pH 1 with 6 N hydrochloric acid. The aqueous layer was separated and 30 ml of ethyl acetate was added thereto. The resulting mixture was adjusted to pH 9.5 with potassium carbonate. The organic layer was separated, washed with water and a saturated aqueous sodium chloride solution in this order, and dried over anhydrous magnesium sulfate. The solvent was removed by distillation under reduced pressure. The residue thus obtained was purified by column chromatography (eluant: chloroform/ethanol = 50/1) to obtain 0.56 g of 1-(benzo[b]furan-5-yl)-2-(pyridin-3-yl methxoy)ethanol (compound No. 28).

Melting point: 85-86 °C [IPE-EtOH]

The compounds shown in Table 6 were obtained in the same manner.

In Table 6, $R^1$, $R^2$, $R^3$, $R^4$, $R^6$ and n each show a substituent or integer used in the following formula:

$$R^1-\underset{\displaystyle OR^2}{\underset{|}{CH}}-\overset{\displaystyle R^3}{\overset{|}{CH}}-O-\left(\overset{\displaystyle R^4}{\overset{|}{CH}}\right)_n-R^6$$

Table 6

| Compound No. | $R^1$ | $R^2$ | $R^3$ | $R^4$ | $R^6$ | n | Addition salt | Melting point (°C) |
|---|---|---|---|---|---|---|---|---|
| 29 | 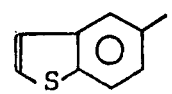 | H | H | H | 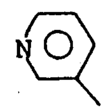 | 1 | - | 106.5-107.5 [AcOEt-IPE] |
| 30 | 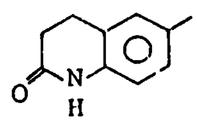 | " | " | " | " | " | HCl | Oily |
| 31 | 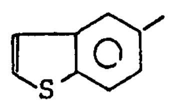 | " | " | " | 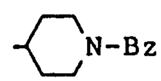 | 2 | - | " |

Table 6 (Cont'd)

| | | | | | | | | |
|---|---|---|---|---|---|---|---|---|
| 32 | 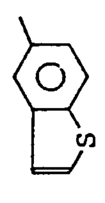 | H | H | H | 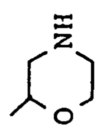 | 1 | - | 121.5-125 [AcOEt] |
| 33 | " | " | " | " | 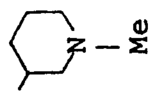 | " | " | 127-129.5 [EtOH-IPE] |
| 34 | " | " | " | " | 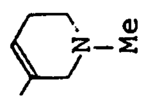 | " | " | 95.5-98 [AcOEt] |
| 35 | " | " | " | - | 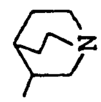 | 0 | HCl | 181-185 [EtOH-AcOEt] |

Production Example 3

(1) A mixture of 5.7 g of potassium tert-butoxide and 57 ml of ethylene glycol was heated to 80°C. Thereto was added, in 1.5 hours, a solution of 18 g of 2-(benzo[b]thiophen-5-yl)oxirane dissolved in 30

ml of dimethyl sulfoxide. The resulting mixture was stirred at the same temperature for 30 minutes. The reaction mixture was added to a mixture of 120 ml of ice water and 80 ml of ethyl acetate. The organic layer was separated. The aqueous layer was extracted twice each with 30 ml of ethyl acetate. The extracts were combined with the previously separated organic layer. The combined organic layer was washed with water and a saturated aqueous sodium chloride solution in this order, and dried over anhydrous magnesium sulfate. The solvent was removed by distillation under reduced pressure. The residue was purified by column chromatography (eluant: chloroform/ethanol = 20/1) to obtain 9.1 g of 1-(benzo[b]thiophen-5-yl)-2-(2-hydroxyethoxy)ethanol.

Melting point: 119-120.5 °C [EtOH-AcOEt]

(2) In 54 ml of pyridine was dissolved 9.0 g of 1-(benzo[b]thiophen-5-yl)-2-(2-hydroxyethoxy)ethanol. To the solution was added, at -25 °C, 7.2 g of p-toluenesulfonyl chloride. The mixture was allowed to stand at 0-5 °C for 24 hours and further at room temperature for 4 hours. The reaction mixture was added to a mixture of 103 ml of 6 N hydrochloric acid, 50 ml of ice water and 100 ml of diethyl ether. The resulting mixture was adjusted to pH 2.0 with 6 N hydrochloric acid. The organic layer was separated. The aqueous layer was extracted with 30 ml of diethyl ether. The extract was combined with the previously separated organic layer. The combined organic layer was washed with water and a saturated aqueous sodium chloride solution in this order, and dried over anhydrous magnesium sulfate. The solvent was removed by distillation under reduced pressure. The residue was purified by column chromatography (eluant: toluene/ethyl acetate = 10/1) to obtain 7.7 g of colorless oily 1-(benzo[b]thiophen-5-yl)-2-[2-(p-toluenesulfonyloxy)ethoxy]ethanol.

(3) 0.97 g of pyridinium p-toluenesulfonate was added, at room temperature, to a solution of 7.6 g of 1-(benzo[b]thiophen-5-yl)-2-[2-(p-toluenesulfonyloxy)ethoxy]ethanol and 3.5 ml of 3,4-dihydro-2H-pyran dissolved in 40 ml of methylene chloride. The mixture was stirred at the same temperature for 20 minutes and further at 40-45 °C for 30 minutes. The reaction mixture was washed with water and dried over anhydrous magnesium sulfate. The solvent was removed by distillation under reduced pressure to obtain 8.7 g of colorless, oily 1-(benzo[b]-thiophen-5-yl)-1-(2-tetrahydropyranyloxy)-2-[2-(p-toluenesulfonyloxy)-ethoxy]ethane.

(4) In 15 ml of ethanol was dissolved 1.5 g of 1-(benzo[b]thiophen-5-yl)-1-(2-tetrahydropyranyloxy)-2-[2-(p-toluenesulfonyloxy)ethoxy]ethane. To the solution was added 4.9 ml of a 40% aqueous methylamine solution. The resulting mixture was refluxed for 1 hour. The reaction mixture was added to a mixture of 20 ml of ice water and 20 ml of diethyl ether. The organic layer was separated. The aqueous layer was extracted with 20 ml of diethyl ether. The extract was combined with the previously separated organic layer. To the combined organic layer was added 20 ml of water. The resulting mixture was adjusted to pH 1.5 with 6 N hydrochloric acid and stirred at room temperature for 20 minutes. The aqueous layer was separated. The organic layer was extracted with 10 ml of water. The extract was combined with the previously separated aqueous layer. To the combined aqueous layer was added 30 ml of methylene chloride. The resulting mixture was adjusted to pH 11 with a 10% aqueous sodium hydroxide solution. The organic layer was separated. The aqueous layer was extracted with 15 ml of methylene chloride. The extract was combined with the previously separated organic layer and the combined organic layer was dried over anhydrous magnesium sulfate. The solvent was removed by distillation under reduced pressure. The residue thus obtained was dissolved in 7 ml of acetone. To the solution was added 0.5 ml of a 5 N dry hydrogen chloride-ethanol solution. The resulting mixture was stirred at room temperature for 1 hour. To the reaction mixture was added 7 ml of diethyl ether. The resulting crystals were collected by filtration to obtain 0.5 g of 1-(benzo[b]thiophen-5-yl)-2-(N-methylaminoethoxy)ethanol hydrochloride (compound No. 36).

Melting point: 201.5-202.5 °C [EtOH-$Me_2CO$]

The compounds shown in Table 7 were obtained in the same manner.

In Table 7, $R^1$, $R^2$, $R^3$, $R^4$, $R^6$ and n each show a substituent or integer used in the following formula:

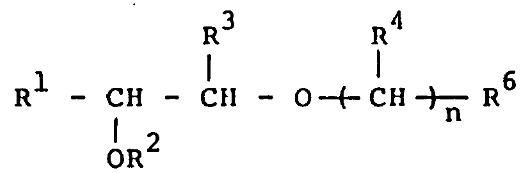

Table 7

| Compound No. | $R^1$ | $R^2$ | $R^3$ | $R^4$ | $R^6$ | n | Addition salt | Melting point (°C) |
|---|---|---|---|---|---|---|---|---|
| 37 | 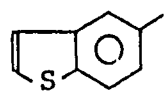 | H | H | H | 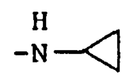 | 2 | HCl | 196.5-197.5 [EtOH-$Me_2CO$] |
| 38 | " | " | " | " | 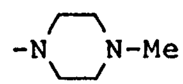 | " | 2HCl | 232-234 [MeOH-$Me_2CO$] |
| 39 | " | " | " | " | 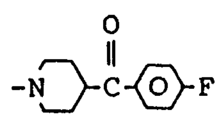 | " | HCl | 219.5-220 [EtOH-AcOEt] |

Table 7 (Cont'd)

| | | | | | | | | |
|---|---|---|---|---|---|---|---|---|
| 40 | 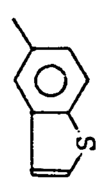 | H | H | H | 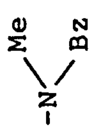 | 2 | Oxalic acid | 138-149 [EtOH-AcOEt] |
| 41 | " | " | " | " | 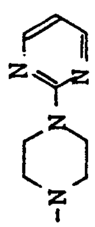 | " | HCl | 170.5-171.5 [EtOH-AcOEt] |
| 42 | " | " | " | " | 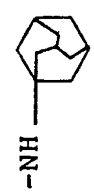 | " | " | 222.5-223 [EtOH-AcOEt] |

Production Example 4

(1) The same procedure as in Production Example 2 was repeated, except that the 2-(benzo[b]furan-5-yl)oxirane and the 3-pyridinemethanol were replaced by 2-(benzo[b]thiophen-5-yl)oxirane and 1,4-diformyl-2-piperazinemethanol, respectively, to obtain oily 1-(benzo[b]thiophen-5-yl)-2-[(1,4-diformylpiperazin-2-yl)methoxy]ethanol (compound No. 43).

(2) In 1.5 ml of methanol was dissolved 270 mg of 1-(benzo[b]thiophen-5-yl)-2-[(1,4-diformylpiperazin-2-yl)methoxy]ethanol. To the solution was added 1.5 ml of a 5 N dry hydrogen chloride-ethanol solution. The resulting mixture was allowed to stand at room temperature overnight. The resulting crystals were collected by filtration, washed with ethanol, and dried to obtain 150 mg of 1-(benzo[b]thiophen-5-yl)-2-[-(piperazin-2-yl)methoxy]ethanol dihydrochloride (compound No. 44).

Melting point: 216-218 °C (decomp.)

Production Example 5

(1) A mixture of 10 g of 2-(N-tritylamino)ethanol, 3.7 g of potassium tert-butoxide and 30 ml of dimethyl sulfoxide was heated to 85 °C. Thereto was added a solution of 5.8 g of 2-(benzo[b]thiophen-5-yl)oxirane dissolved in 10 ml of dimethyl sulfoxide. The resulting mixture was stirred at the same temperature for 5 minutes. The reaction mixture was added to a mixture of 150 ml of ice water and 100 ml of ethyl acetate. The organic layer was separated. The aqueous layer was extracted with 30 ml of ethyl acetate. The extract was combined with the previously separated organic layer. The combined organic layer was washed with water and a saturated aqueous sodium chloride solution in this order, and dried over anhydrous magnesium sulfate. The solvent was removed by distillation under reduced pressure. To the residue thus obtained were added 70 ml of a 50% aqueous formic acid solution and 30 ml of tetrahydrofuran. The resulting mixture was stirred at 50-60 °C for 1 hours. The solvent was removed by distillation under reduced pressure. To the residue thus obtained were added 50 ml of ethyl acetate and 30 ml of water. The resulting mixture was adjusted to pH 2 with 6 N hydrochloric acid. The aqueous layer was separated. The organic layer was extracted twice each with 10 ml of water. The extracts were combined with the previously separated aqueous layer. To the combined aqueous layer was added 50 ml of methylene chloride and the resulting mixture was adjusted to pH 10.5 with potassium carbonate. The organic layer was separated, washed with water, and dried over anhydrous magnesium sulfate. The solvent was removed by distillation under reduced pressure to obtain 1.2 g of 1-(benzo[b]thiophen-5-yl)-2-(2-aminoethoxy)ethanol (compound No. 45).

Melting point: 87-90.5 °C [EtOH-IPE]

(2) 1.1 g of 1-(benzo[b]thiophen-5-yl)-2-(2-aminoethoxy)ethanol was dissolved in 10 ml of ethanol. To the solution was added 290 mg of fumaric acid. The resulting mixture was stirred at room temperature for 30 minutes. To the reaction mixture was added 7 ml of diethyl ether. The resulting mixture was stirred at the same temperature for 1 hour. The resulting crystals were collected by filtration and dried to obtain 1.2 g of 1-(benzo[b]thiophen-5-yl)-2-(2-aminoethoxy)ethanol 1/2 fumarate (compound No. 46).

Melting point: 204.5-205.5 °C [MeOH-EtOH]

Production Example 6

The same procedure as in Production Example 5 was repeated, except that the 2-(N-tritylamino)ethanol was replaced by (1-tritylimidazol-4-yl)methanol, to obtain 1-(benzo[b]thiophen-5-yl)-2-[(imidazolyl)methoxy]-ethanol (compound No. 47) having a melting point of 128-129 °C [AcOEt].

In the above name of the compound obtained, the term "(imidazolyl)methoxy" was used because it is not clear yet to which carbon of the 4- and 5-position carbons of the imidazolyl group the carbon of the methoxy group bonds.

Production Example 7

0.46 g of 1-(benzo[b]thiophen-5-yl)-2-(2-aminoethoxy)ethanol was dissolved in a mixture of 5 ml of water and 5 ml of dioxane. Thereto was added 0.21 g of sodium carbonate. The resulting mixture was heated to 50 °C. Thereto was added 0.22 g of 2-chloropyrimidine. The resulting mixture was refluxed for 3 hours. The reaction mixture was added to a mixture of 30 ml of ice water and 30 ml of ethyl acetate. The organic layer was separated. The aqueous layer was extracted with 10 ml of ethyl acetate. The extract was combined with the previously separated organic layer. To the combined organic layer was added 20 ml of water and the resulting mixture was adjusted to pH 1.5 with 6 N hydrochloric acid. The aqueous layer was separated. The organic layer was extracted with 10 ml of water. The extract was combined with the previously separated aqueous layer. To the combined aqueous layer was added 50 ml of methylene chloride and the resulting mixture was adjusted to pH 10.5 with potassium carbonate. The organic layer was separated, washed with water, and dried over anhydrous magnesium sulfate. The solvent was removed by

distillation under reduced pressure. The residue thus obtained was purified by column chromatography (eluant: chloroform/ethanol = 20/1) to obtain an oily product. To the oily product were added 2 ml of ethanol and 70 mg of maleic acid. The resulting mixture was stirred at room temperature for 1 hour. To the reaction mixture was added 2 ml of diethyl ether. The resulting crystals were collected by filtration and dried to obtain 0.28 g of 1-(benzo[b]-thiophen-5-yl)-2-{[2-(pyrimidin-2-yl)amino]ethoxy} ethanol 1/2 maleate (compound No. 48).

Melting point: 113.5-114.5 °C [IPA-AcOEt]

Production Example 8

0.39 g of N,N'-dicyclohexylcarbodiimide was added, with ice cooling, to a mixture of 0.45 g of 1-(benzo-[b]thiophen-5-yl)-2-(2-aminoethoxy)ethanol, 0.23 g of nicotinic acid, 0.26 g of 1-hydroxybenzotriazole, 0.26 ml of triethylamine and 3 ml of tetrahydrofuran. The resulting mixture was stirred at the same temperature for 5 minutes and further at room temperature for 2 hours. To the reaction mixture were added 20 ml of water and 20 ml of ethyl acetate. The insolubles were removed by filtration. The filtrate was adjusted to pH 1.5 with 6 N hydrochloric acid. The aqueous layer was separated. The organic layer was extracted twice each with 5 ml of water. The extracts were combined with the previously separated aqueous layer. To the combined aqueous layer was added 30 ml of chloroform and the resulting mixture was adjusted to pH 10.5 with potassium carbonate. The organic layer was separated, washed with water, and dried over anhydrous magnesium sulfate. The solvent was removed by distillation under reduced pressure. The residue thus obtained was purified by column chromatography (eluant: chloroform/ethanol = 10/1). The resulting oily product was dissolved in 3 ml of ethanol. To the solution was added 0.24 ml of a 5 N dry hydrogen chlorideethanol solution. The resulting mixture was stirred at room temperature for 1 hour. To the reaction mixture was added 1.5 ml of diethyl ether. The resulting mixture was stirred at the same temperature for 1 hour. The resulting crystals were collected by filtration and dried to obtain 0.31 g of 1-(benzo[b]thiophen-5-yl)-2-[2-(nicotinoylamino)ethoxy]ethanol hydrochloride (compound No. 49).

Melting point: 152-153 °C [EtOH-AcOEt]

Production Example 9

(1) 1.6 g of 4-methyl-2-formylthiazole was dissolved in 30 ml of tetrahydrofuran. The solution was cooled to -30 °C. Thereto was dropwise added, in 10 minutes, 10 ml of a tetrahydrofuran solution containing 1.6 M of 2-chloroethoxymethylmagnesium chloride. The mixture was stirred for 1 hour with ice cooling. The reaction mixture was added to a mixture of 50 ml of ice water, 50 ml of ethyl acetate and 2 g of ammonium chloride. The resulting mixture was adjusted to pH 2 with 6 N hydrochloric acid and stirred at the same temperature for 5 minutes. The reaction mixture was adjusted to pH 6 with a saturated aqueous sodium hydrogencarbonate solution. The organic layer was separated, washed with water and a saturated aqueous sodium chloride solution in this order, and dried over anhydrous magnesium sulfate. The solvent was removed by distillation under reduced pressure. The residue thus obtained was purified by column chromatography (eluant: toluene/ethyl acetate = 4/1) to obtain 1.3 g of oily 1-(4-methyl-2-thiazolyl)-2-(2-chloroethoxy) ethanol.

(2) A mixture of 1.2 g of 1-(4-methyl-2-thiazolyl)-2-(2-chloroethoxy)ethanol, 3 ml of a 50% aqueous dimethylamine solution, 0.45 g of potassium iodide and 20 ml of ethanol was refluxed for 3 hours. To the reaction mixture was added 3 ml of a 50% aqueous dimethylamine solution. The resulting mixture was refluxed for 3 hours. The solvent was removed by distillation under reduced pressure. To the residue thus obtained were added 30 ml of ethyl acetate and 30 ml of water. The resulting mixture was adjusted to pH 1.5 with 6 N hydrochloric acid. The aqueous layer was separated and washed with 10 ml of ethyl acetate. Thereto was added 30 ml of ethyl acetate. The resulting mixture was adjusted to pH 10.5 with potassium carbonate. The organic layer was separated, washed with 10 ml of water and 10 ml of a saturated aqueous sodium chloride solution in this order, and dried over anhydrous magnesium sulfate. The solvent was removed by distillation under reduced pressure. The residue thus obtained was dissolved in 6 ml of ethanol. To the solution were added 0.6 ml of a 5 N dry hydrogen chloride-ethanol solution and 6 ml of diethyl ether. The mixture was stirred at room temperature for 1 hour. The resulting crystals were collected by filtration, washed with 2 ml of a 1:1 mixture of diethyl ether and ethanol, and dried to obtain 390 mg of 1-(4-methyl-2-thiazolyl)-2-[2-(N,N-dimethylamino)ethoxy]ethanol hydrochloride (compound No. 50).

Melting point: 159-160 °C [IPA-AcOEt]

The compounds shown in Table 8 were obtained in the same manner.

In Table 8, $R^1$, $R^2$, $R^3$, $R^4$, $R^6$ and n each show a substituent or integer used in the following formula:

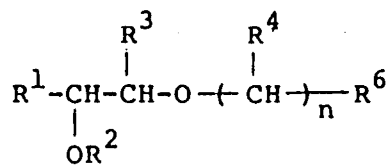

Table 8

| Compound No. | $R^1$ | $R^2$ | $R^3$ | $R^4$ | $R^6$ | n | Addition salt | Melting point (°C) |
|---|---|---|---|---|---|---|---|---|
| 51 | 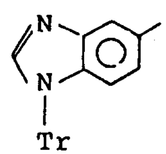 | H | H | H | 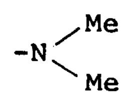 | 2 | - | Oily |
| 52 *1 | 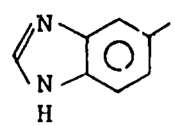 | " | " | " | " | " | 2HCl | 185-186.5 [EtOH-AcOEt] |
| 53 | 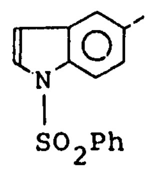 | " | " | " | " | " | - | Oily |

Table 8 (Cont'd)

| | | | | | | | | |
|---|---|---|---|---|---|---|---|---|
| 54 *2 | 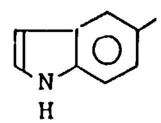 | H | H | H | 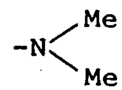 | 2 | - | Oily |
| 55 | 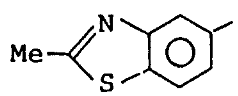 | " | " | " | " | " | HCl | 175-176 [EtOH-AcOEt] |
| 56 | 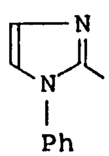 | " | " | " | " | " | 2HCl | Oily |
| 57 | 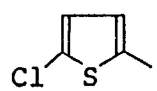 | " | " | " | " | " | HCl | 182.5-183 [EtOH-AcOEt] |

Table 8 (Cont'd)

| | | | | | | | | |
|---|---|---|---|---|---|---|---|---|
| 54 *2 | 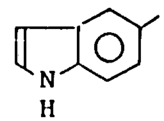 | H | H | H | 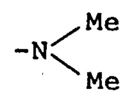 | 2 | - | Oily |
| 55 | 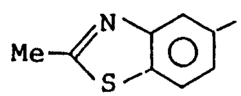 | " | " | " | " | " | HCl | 175-176 [EtOH-AcOEt] |
| 56 | 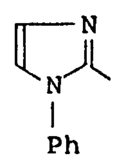 | " | " | " | " | " | 2HCl | Oily |
| 57 | 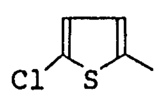 | " | " | " | " | " | HCl | 182.5-183 [EtOH-AcOEt] |

Table 8 (Cont'd)

| | | | | | | | | |
|---|---|---|---|---|---|---|---|---|
| 62 | 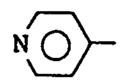 | H | H | H | 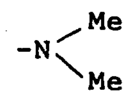 | 2 | 2HCl | 184-186 [EtOH-AcOEt] |
| 63 | 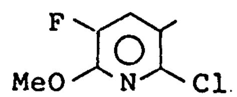 | " | " | " | " | " | HCl | 196-197 [MeOH] |
| 64 | 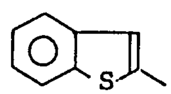 | " | " | " | 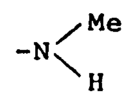 | " | " | 193-193.5 [EtOH-AcOEt] |
| 65*3 | 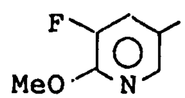 | " | " | " | 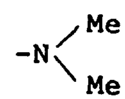 | " | " | 162-163 [IPA] |

Table 8 (Cont'd)

| | | | | | | | | |
|---|---|---|---|---|---|---|---|---|
| 66 | 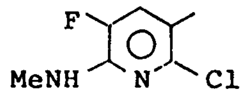 | H | H | H | 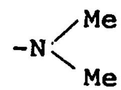 | 2 | HCl | 153.5-154 [IPA] |
| 67 | 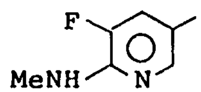 | " | " | " | " | " | 2HCl | 176-179 |
| 68 | 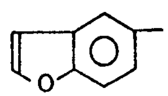 | " | " | " | 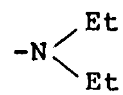 | " | - | Oily |
| 69 | 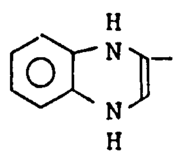 | " | " | " | 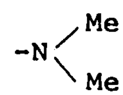 | " | " | " |

Table 8 (Cont'd)

| | | | | | | | | |
|---|---|---|---|---|---|---|---|---|
| 70 | 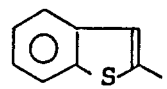 | H | H | H | 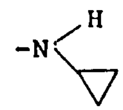 | 2 | HCl | 194.5-195 [EtOH-AcOEt] |
| 71 | 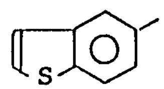 | " | " | " | 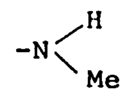 | 3 | - | 109-111 [AoOEt] |
| 72 | " | " | " | " | 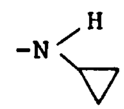 | " | HCl | 133.5-134.5 [EtOH-AcOEt] |
| 73 | " | " | " | " | 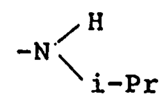 | " | " | 136.5-139.5 [EtOH-AcOEt] |

Table 8 (Cont'd)

| | | | | | | | |
|---|---|---|---|---|---|---|---|
| 74 | 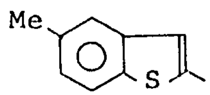 | H | H | H | 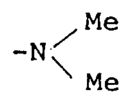 | 2 | HCl | 193-193.5 [EtOH-AcOEt] |
| 75 | 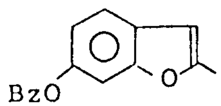 | " | " | " | " | " | " | 171.5-172 [EtOH-AcOEt] |
| 76 | 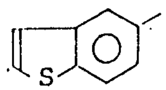 | " | " | " | 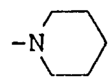 | 3 | " | 137.5-139.5 [EtOH-AcOEt] |
| 77 | " | " | " | " | 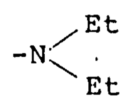 | 2 | " | 138.5-139 [EtOH-AcOEt] |

Table 8 (Cont'd)

| | | | | | | | | |
|---|---|---|---|---|---|---|---|---|
| 78 | 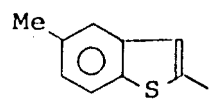 | H | H | H | 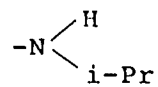 | 2 | HCl | 184-184.5 [EtOH-AcOEt] |
| 79 | 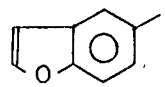 | " | " | " | 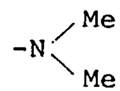 | 3 | - | 68.5-69.5 [Hexane] |
| 80 | 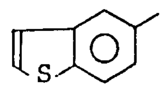 | " | " | " | 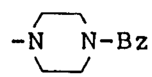 | 2 | 2HCl | 250-252.5 (decomp.) [MeOH-$H_2O$] |
| 81 | " | " | " | " | 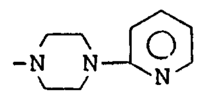 | " | " | 155-157 [EtOH] |

Table

Table 8 (Cont'd)

| | | | | | | | | |
|---|---|---|---|---|---|---|---|---|
| 82 | 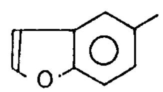 | H | H | H | 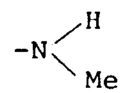 | 3 | - | 65-67.5 [IPA-IPE] |
| 83 | " | " | " | " | 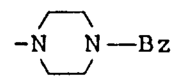 | 2 | 2HCl | 234-234.5 [MeOH-AcOEt] |
| 84 | " | " | " | " | 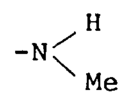 | " | HCl | 178-180.5 [IPA-AcOEt] |
| 85 | 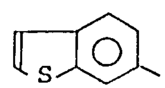 | " | " | " | 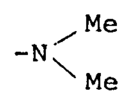 | 3 | - | 52-53 [IPE] |

Table 8 (Cont'd)

| | | | | | | | | |
|---|---|---|---|---|---|---|---|---|
| 86 | 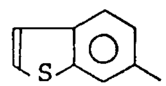 | H | H | H | 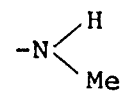 | 3 | - | 81.5-83 [IPA-IPE] |
| 87 | " | " | " | " | " | 2 | HCl | 196-198 [EtOH-AcOEt] |
| 88 | " | " | " | " | 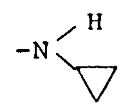 | " | " | 190-192.5 [EtOH-AcOEt] |
| 89 | 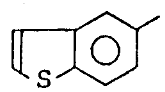 | " | " | " | 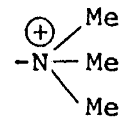 | " | 1/2 1,5-naphthalene-disulfonic acid | Amorphous |

Table 8 (Cont'd)

| | | | | | | | | |
|---|---|---|---|---|---|---|---|---|
| 90 | 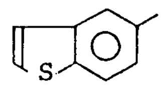 | Ac | H | H | 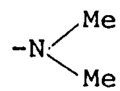 | 2 | HCl | Oily |
| 91 | 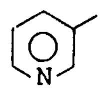 | H | " | " | 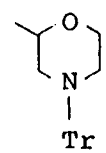 | 1 | — | " |
| 92 | " | " | " | " | 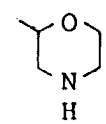 | " | 1/2 Maleic acid | 110-117 |

Note:
*1: This compound can be obtained by subjecting compound No. 51 to hydrolysis reaction using hydrochloric acid.

*2: This compound can be obtained by subjecting compound No. 53 to hydrolysis reaction using sodium hydroxide.

*3: This compound can be obtained by subjecting compound No. 63 to conventional hydrogenation reaction.

Production Example 10

(1) A mixture of 9.2 g of 1-(2-thienyl)-2-[2-(N,N-dimethylamino)ethoxy]ethanol and 18 ml of acetic anhydride was refluxed for 10 minutes. The reaction mixture was dropwise added to a mixture of 7.8 ml of concentrated nitric acid and 27 ml of acetic anhydride at 0 °C in 30 minutes. The resulting mixture

was stirred at the same temperature for 2 hours. The reaction mixture was added to a saturated aqueous sodium hydrogen-carbonate solution with the pH of the resulting mixture having been adjusted to 7 with a 40% aqueous sodium hydroxide solution. The resulting mixture was adjusted to pH 10 with a 40% aqueous sodium hydroxide solution and 300 ml of chloroform was added thereto. The organic layer was separated and 300 ml of water was added thereto. The resulting mixture was adjusted to pH 2 with 6 N hydrochloric acid. The aqueous layer was separated and 300 ml of chloroform was added thereto. The resulting mixture was adjusted to pH 10 with a 40% aqueous sodium hydroxide solution. The organic layer was separated, washed with water, and dried over anhydrous magnesium sulfate. The solvent was removed by distillation under reduced pressure to obtain 10.4 g of oily 1-(5-nitro-2-thienyl)-1-acetoxy-2-[2-(N,N-dimethylamino)ethoxy]ethane (compound No. 93).

(2) In 10 ml of methanol was dissolved 320 mg of 1-(5-nitro-2-thienyl)-1-acetoxy-2-[2-(N,N-dimethylamino)ethoxy]ethane. To the solution was added 1.27 ml of a 1 N aqueous sodium hydroxide solution. The resulting mixture was stirred at room temperature for 1 hour. To the reaction mixture were added 40 ml of chloroform and 40 ml of water. The organic layer was separated and 30 ml of water was added thereto. The resulting mixture was adjusted to pH 2 with 6 N hydrochloric acid. The aqueous layer was separated and 30 ml of chloroform was added thereto. The resulting mixture was adjusted to pH 11 with a 10% aqueous sodium hydroxide solution. The organic layer was separated, washed with water, and dried over anhydrous magnesium sulfate. The solvent was removed by distillation under reduced pressure. To the residue thus obtained were added 3 ml of methanol and 1 ml of a 5 N dry hydrogen chloride-ethanol solution. The solvent was removed by distillation under reduced pressure. To the residue thus obtained was added 5 ml of ethanol. The resulting crystals were collected by filtration and dried to obtain 170 mg of 1-(5-nitro-2-thienyl)-2-[2-(N,N-dimethylamino)ethoxy]ethanol (compound No. 94).

Melting point: 189-191.5 °C (decomp.)

Production Example 11

(1) In 10 ml of pyridine was dissolved 3.4 g of 2-[2-(N,N-dimethylamino)ethoxy]-1-(6-benzyloxybenso[b]-furan-2-yl)ethanol. To the solution was added 1.8 ml of acetic anhydride. The resulting mixture was stirred at room temperature for 17.5 hours. The solvent was removed by distillation under reduced pressure. To the residue thus obtained were added 40 ml of ethyl acetate and 40 ml of water. The resulting mixture was adjusted to pH 7 with sodium hydrogencarbonate. The organic layer was separated. The aqueous layer was extracted with 20 ml of ethyl acetate. The extract was combined with the previously separated organic layer. The combined organic layer was washed with water and a saturated aqueous sodium chloride solution in this order, and dried over anhydrous magnesium sulfate. The solvent was removed by distillation under reduced pressure. The residue thus obtained was purified by column chromatography (eluant: chloroform/ethanol = 1/1) to obtain 3.25 g of oily 1-acetoxy-1-(6-benzyloxybenzo [b] furan-2-yl)-2-[2-(N,N-dimethylamino)ethoxy] ethane (compound No. 95).

IR (neat) $cm^{-1}$: $\nu_{C=O}$ 1740

(2) A mixture of 3.2 g of 1-acetoxy-1-(6-benzyloxybenzo[b]furan-2-yl)-2-[2-(N,N-dimethylamino)ethoxy]-ethane, 0.6 g of 5% palladium-carbon, 0.67 ml of concentrated hydrochloric acid and 30 ml of methanol was subjected to hydrogenation at room temperature under atmospheric pressure for 1.5 hours. After the completion of the reaction, the palladium-carbon was removed by filtration. The solvent was removed by distillation under reduced pressure. To the residue thus obtained were added 20 ml of chloroform and 20 ml of water. The resulting mixture was adjusted to pH 7 with sodium hydrogencarbonate. The organic layer was separated. The aqueous layer was extracted with 10 ml of chloroform. The extract was combined with the previously separated organic layer. The combined organic layer was washed with 5 ml of water, and dried over anhydrous magnesium sulfate. The solvent was removed by distillation under reduced pressure. The residue thus obtained was purified by column chromatography (eluant: chloroform/methanol = 7/1) to obtain 1.57 g of oily 1-acetoxy-1-(6-hydroxybenzo[b]furan-2-yl)-2-[2-(N,N-dimethylamino)ethoxy]ethane (compound No. 96).

IR (neat) $cm^{-1}$: $\nu_{C=O}$ 1740

(3) In 3.5 ml of benzene was dissolved 0.65 g of 1-acetoxy-1-(6-hydroxybenzo[b]furan-2-yl)-2-[2-(N,N-dimethylamino)ethoxy]ethane. To the solution was added 0.33 ml of ethyl isocyanate. The resulting mixture was stirred for 30 minutes at 80 °C. The solvent was removed by distillation under reduced pressure. The residue thus obtained was purified by column chromatography (eluant: chloroform/ethanol = 6/1) to obtain an oily product. The oily product was treated with dry hydrogen chloride according to a conventional method to obtain 0.58 g of oily 1-acetoxy-1-(6-N-ethylcarbamoyloxybenzo[b]furan-2-yl)-2-[2-(N,N-dimethylamino)ethoxy]ethane hydrochloride (compound No. 97).

IR (neat) $cm^{-1}$: $\nu_{C=O}$ 1730

Next, this invention is specifically described by way of Preparation Examples. However, this invention is in no way restricted to these Examples.

Preparation Example 1 (Tablets)

Tablets each containing 50 mg of 2-[2-N,N-dimethylamino)ethoxy]-1-(benzo[b]thiophen-5-yl)ethanol hydrochloride (compound No. 1) were prepared using the following recipe according to the following method:

| Per tablet: | | |
|---|---|---|
| Compound No. 1 | 50 mg | ① |
| Lactose | 20 mg | ① |
| Kollidon CL (BASF's product) | 15 mg | ① |
| Corn starch | 30 mg | ① |
| AVICEL PH 101 (Asahi Kasei's product) | 50 mg | ① |
| Polyvinylpyrrolidone K-90 | 5 mg | |
| Light silica | 3 mg | ② |
| Magnesium stearate | 2 mg | ② |
| Total | 175 mg | |

The components ① were kneaded with an aqueous solution containing 8% of Polyvinylpyrrolidone K-90. The kneaded product was dried at 40 °C and mixed with the components ②. The resulting mixture was made into round tablets each weighing 175 mg and having a diameter of 8 mm.

Preparation Example 2 (Capsules)

Capsules each containing 50 mg of 2-[(N-methyl-1H-1,2,5,6-tetrahydropyridin-3-yl)methyl]-1-(benzo[b]-thiophen-5-yl)ethanol (compound No. 34) were prepared using the following recipe according to the following method:

Per capsule:

| | | |
|---|---|---|
| Compound No. 34 | 50 mg | ① |
| Lactose | 20 mg | ① |
| Corn starch | 53 mg | ① |
| Kollidon CL (BASF's product) | 2 mg | ① |
| Polyvinylpyrrolidone K-90 | 5 mg | |
| AVICEL PH 302 (Asahi Kasei's product) | 18 mg | ② |
| Magnesium stearate | 2 mg | ② |
| Total | 150 mg | |

The components ① were kneaded with an aqueous solution containing 8% of Polyvinylpyrrolidone K-90. The kneaded product was dried at 40 °C and mixed with the components ②. The resulting mixture was charged into No. 3 gelatin capsules in an amount of 150 mg per capsule to obtain capsules.

Preparation Example 3 (Tablets)

2-[(N-methyl-1H-1,2,5,6-tetrahydropyridin-3-yl)methyl]-1-(benzo[b]-thiophen-5-yl)ethanol (compound No. 34), 2-(2-aminoethoxy)-1-(benzo[b]thiophen-5-yl)ethanol $\frac{1}{2}$ fumarate (compound No. 46), 2-[2-(N,N-diethylamino)ethoxy]-1-(benzo[b]thiophen-5-yl)ethanol hydrochloride (compound No. 77) and 2-[2-(4-benzylpiperazin-1-yl)ethyl]-1-(benzo[b]furan-5-yl)ethanol dihydrochloride (compound No. 83) were processed as in Preparation Example 1 to obtain tablets each containing 50 mg of one of the above compounds.

Preparation Example 4 (Capsules)

2-[2- (N,N-dimethylamino)ethoxy]-1-(benzo[b]thiophen-5-yl)ethanolhydrochloride (compound No. 1), 2-(2-aminoethoxy)-1-(benzo[b]thiophen-5-yl)ethanol $\frac{1}{2}$ fumarate (compound No. 46), 2-[2-(N,N-diethylamino)-ethoxy]-1-(benzo[b]thiophen-5-yl)ethanol hydrochloride (compound No. 77) and 2-[2-(4-benzylpiperazin-1-yl)ethyl]-1-(benzo[b]furan-5-yl)ethanol dihydrochloride (compound No. 83) were processed as in Preparation Example 2 to obtain capsules each containing 50 mg of one of the above compounds.

## Claims

**1.** A 1,2-ethanediol derivative represented by the following general formula or a salt thereof:

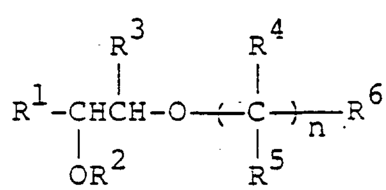

wherein $R^1$ represents a substituted or unsubstituted heterocyolic group; $R^2$ represents a hydrogen atom, a $C_{1-6}$ alkyl group or a hydroxyl-protecting group; $R^3$ represents a hydrogen atom or a $C_{1-6}$ alkyl group; $nR^4$'s and $nR^5$'s are the same as or different from one another and represent hydrogen atoms or $C_{1-6}$ alkyl groups; $R^6$ represents an ammonio group or a substituted or unsubstituted amino

or nitrogen-containing heterocyclic group, the nitrogen-containing heterocyclic group being selected from the group consisting of pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and n represents 0 or an integer of 1 to 6, wherein the substituent on $R^1$ is selected from the group consisting of halogen atoms, substituted or unsubstituted amino, $C_{1-6}$ alkyl, aryl, ar-$C_{1-4}$ alkyl, $C_{1-6}$ alkoxy, ar-$C_{1-4}$-alkoxy, aryloxy, carbamoyloxy, $C_{1-6}$ alkylthio, $C_{2-6}$-alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino and heterocyclic groups, protected amino groups, protected or unprotected hydroxyl groups, nitro group, oxo group and $C_{1-4}$ alkylenedioxy groups; the substituted $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected or unprotected hydroxyl groups, protected or unprotected amino groups, protected or unprotected carboxyl groups, unsubstituted $C_{1-6}$ alkyl groups, $C_{1-6}$ alkyl groups substituted by a protected or unprotected hydroxyl group, unsubstituted or halogen-substituted aryl groups, unsubstituted or halogen-substituted aroyl groups, unsubstituted $C_{1-6}$ alkoxy groups, $C_{1-6}$ alkoxy groups substituted by a $C_{1-6}$ alkoxy group, $C_{1-6}$ acyl groups, ar-$C_{1-4}$ alkyl groups, ar-$C_{2-6}$ alkenyl groups, heterocyclic groups, heterocyclic-CO- groups, oxo group, $C_{1-6}$ alkylsulfonyl groups and arylsulfonyl groups; and the substituted amino group as the substituent of $R^1$ and the substituted amino group as $R^6$ have each at least one substituent selected from the group consisting of protected or unprotected hydroxyl groups, unsubstituted $C_{1-6}$ alkyl groups, $C_{1-6}$ alkyl groups substituted by a protected or unprotected carboxyl or hydroxyl group, cycloalkyl groups, aryl groups, $C_{1-6}$ acyl groups, ar-$C_{1-4}$ alkyl groups, heterocyclic groups, unsubstituted or oxo-substituted heterocyclic-CO- groups, adamantyl group, $C_{1-6}$ alkylsulfonyl groups and arylsulfonyl groups; all the above heterocyclic groups being selected from the group consisting of the nitrogen-containing heterocyclic groups mentioned in the definition of $R^6$ and furyl, thienyl, benzothienyl, pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo[2,3-a]pyridyl, benzo[b]piperazinyl, chromenyl, isothiazolyl, isoxazolyl, oxadiazolyl, pyridazinyl, isoindolyl and isoquinolyl groups.

**2.** A 1,2-ethanediol derivative or a salt thereof according to Claim 1, wherein $R^1$ represents a benzothienyl, benzofuranyl or 2,3-dihydrobenzothienyl group; $R^2$ represents a hydrogen atom; $R^3$ represents a hydrogen atom; $nR^4$'s and $nR^5$'s represent hydrogen atoms; $R^6$ represents a pyridyl group, a piperidinyl group which may optionally be substituted by a $C_{1-6}$ alkyl group, a 1,2,5,6-tetrahydropyridyl group which may optionally be substituted by a $C_{1-6}$ alkyl group, an imidazolyl group, a phenyl-$C_{1-4}$alkyl group-substituted piperazinyl group or an amino group which may optionally be substituted by a $C_{1-6}$ alkyl or cycloalkyl group; and n represents an integer of 1 to 4.

**3.** 1-(Benzo[b]thiophen-5-yl)-2-[2-(N,N-dimethylamino)ethoxy]ethanol or a salt thereof.

**4.** 1-(Benzo[b]thiophen-5-yl)-2-[3-(N,N-dimethylamino)propoxy]ethanol or a salt thereof.

**5.** 1-(Benzo[b]thiophen-5-yl)-2-(imidazolylmethoxy)ethanol or a salt thereof.

**6.** 1-(Benzo[b]thiophen-5-yl)-2-[(1-methyl-1,2,5,6-tetrahydropyridin-3-yl)methoxy]ethanol or a salt thereof.

**7.** 1-(Benzo[b]furan-5-yl)-2-[2-(N,N-dimethylamino)ethoxy]ethanol or a salt thereof.

**8.** 1-(Benzo[b]thiophen-5-yl)-2-[2-(N,N-diethylamino)ethoxy]ethanol or a salt thereof.

**9.** A process for producing a 1,2-ethanediol derivative represented by the following general formula or a salt thereof:

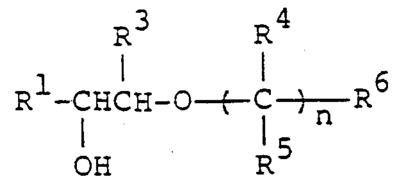

wherein $R^1$ represents a substituted or unsubstituted heterocyclic group; $R^3$ represents a hydrogen atom or a $C_{1-6}$ alkyl group; $nR^4$'s and $nR^5$'s are the same as or different from one another and represent hydrogen atoms or $C_{1-6}$ alkyl groups; $R^6$ represents an ammonio group or a substituted or unsubstituted amino or nitrogen-containing heterocyclic group, the nitrogen-containing heterocyclic group being selected from the group consisting of pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and n represents 0 or an integer of 1 to 6, wherein the substituent on $R^1$ is selected from the group consisting of halogen atoms, substituted or unsubstituted amino, $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino and heterocyclic groups, protected amino groups, protected or unprotected hydroxyl groups, nitro group, oxo group and $C_{1-4}$ alkylenedioxy groups; the substituted $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected or unprotected hydroxyl groups, protected or unprotected amino groups, protected or unprotected carboxyl groups, unsubstituted $C_{1-6}$ alkyl groups, $C_{1-6}$ alkyl groups substituted by a protected or unprotected hydroxyl group, unsubstituted or halogen-substituted aryl groups, unsubstituted or halogen-substituted aroyl groups, unsubstituted $C_{1-6}$ alkoxy groups, $C_{1-6}$ alkoxy groups substituted by a $C_{1-6}$ alkoxy group, $C_{1-6}$ acyl groups, ar-$C_{1-4}$ alkyl groups, ar-$C_2$-$C_6$ alkenyl groups, heterocyclic groups, heterocyclic-CO- groups, oxo group, $C_{1-6}$ alkylsulfonyl groups and arylsulfonyl groups; and the substituted amino group as the substituent of $R^1$ and the substituted amino group as $R^6$ have each at least one substituent selected from the group consisting of protected or unprotected hydroxyl groups, unsubstituted $C_{1-6}$ alkyl groups, $C_{1-6}$ alkyl groups substituted by a protected or unprotected carboxyl or hydroxyl group, cycloalkyl groups, aryl groups, $C_{1-6}$ acyl groups, ar-$C_{1-4}$ alkyl groups, heterocyclic groups, unsubstituted or oxosubstituted heterocyclic-CO- groups, adamantyl group, $C_{1-6}$ alkylsulfonyl groups and arylsulfonyl groups; all the above heterocyclic groups being selected from the group consisting of the nitrogen-containing heterocyclic groups mentioned in the definition of $R^6$ and furyl, thienyl, benzothienyl, pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo[2,3-a]pyridyl, benzo[b]-piperazinyl, chromenyl, isothiazolyl, isoxazolyl, oxadiazolyl, pyridazinyl, isoindolyl and isoquinolyl groups, which comprises reacting a compound represented by the general formula:

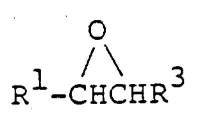

wherein $R^1$ and $R^3$ have the same meanings as defined above, with a compound represented by the following general formula or a salt thereof:

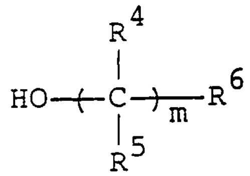

wherein $mR^4$'s and $mR^5$'s are the same as or different from one another and represent hydrogen atoms or lower alkyl groups, $R^6$ has the same meaning as defined above and m represents an integer of 1 to 6, or a compound represented by the following general formula or a salt thereof:

$HO\text{-}R^{6a}$

wherein $R^{6a}$ represents the same substituted or unsubstituted nitrogen-containing heterocyclic group as in the defintion of $R^6$, provided that the heterocyclic group has a free valence on a carbon atom forming the heterocyclic ring.

**10.** A process for producing a 1,2-ethanediol derivative represented by the following general formula or a salt thereof:

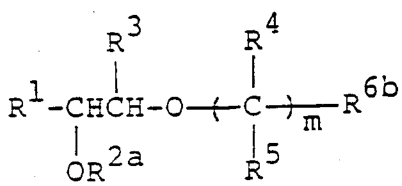

wherein $R^1$ represents a substituted or unsubstituted heterocyclic group; $R^{2a}$ represents a hydroxyl-protecting group; $R^3$ represents a hydrogen atom or a $C_{1-6}$ alkyl group; $mR^4$'s and $mR^5$'s are the same as or different from one another and represent hydrogen atoms or $C_{1-6}$ alkyl groups; $R^{6b}$ represents a substituted or unsubstituted amino or nitrogen-containing heterocyclic group, the nitrogen-containing heterocyclic group having a free valence on a nitrogen atom forming the heterocyclic ring and being selected from the group consisting of pyrrolyl, pyrrolidinyl, piperidyl, piperazinyl, imidazolyl, pyrazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and m represents an integer of 1 to 6, wherein the substituent on $R^1$ is selected from the group consisting of halogen atoms, substituted or unsubstituted amino, $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino and heterocyclic groups, protected amino groups, protected or unprotected hydroxyl groups, nitro group, oxo group and $C_{1-4}$ alkylenedioxy groups; the substituted $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino or heterocyclic group as the substituent of $R^1$ and the substituted nitrogen-containing heterocyclic group as $R^{6b}$ have each at least one substituent selected from the group consisting of halogen atoms, protected or unprotected hydroxyl groups, protected or unprotected amino groups, protected or unprotected carboxyl groups, unsubstituted $C_{1-6}$ alkyl groups, $C_{1-6}$ alkyl groups substituted by a protected or unprotected hydroxyl group, unsubstituted or halogen-substituted aryl groups, unsubstituted or halogen-substituted aroyl groups, unsubstituted $C_{1-6}$ alkoxy groups, $C_{1-6}$ alkoxy groups substituted by a $C_{1-6}$ alkoxy group, $C_{1-6}$ acyl groups, ar-$C_{1-4}$ alkyl groups, ar-$C_{2-6}$ alkenyl groups, heterocyclic groups, heterocyclic-CO-groups, oxo group, $C_{1-6}$ alkylsulfonyl groups and arylsulfonyl groups; and the substituted amino group as the substituent of $R^1$ and the substituted amino group as $R^{6b}$ have each at least one substituent selected from the group consisting of protected or unprotected hydroxyl groups, unsubstituted $C_{1-6}$ alkyl groups, $C_{1-6}$ alkyl groups substituted by a protected or unprotected carboxyl or hydroxyl group, cycloalkyl groups, aryl groups, $C_{1-6}$ acyl groups, ar-$C_{1-4}$ alkyl groups, heterocyclic groups, unsubstituted or oxo-substituted heterocyclic-CO- groups, adamantyl group, $C_{1-6}$ alkylsulfonyl groups and arylsulfonyl groups;
all the above heterocyclic groups being selected from the group consisting of the nitrogen-containing heterocyclic groups mentioned in the definition of $R^{6b}$ and furyl, thienyl, benzothienyl, pyranyl, isobenzofuranyl, oxazolyl, benzofuranyl, indolyl, benzimidazolyl, benzoxazolyl, benzothiazolyl, quinoxalyl dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo[2,3-a]pyridyl, benzo[b]piperazinyl, chromenyl, isothiazolyl, isoxazolyl, oxadiazolyl, pyridazinyl, isoindolyl and isoquinolyl groups, which comprises reacting a compound represented by the general formula:

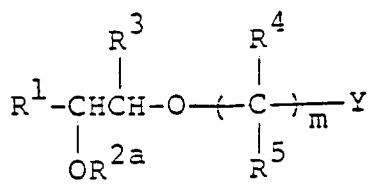

wherein $R^1$, $R^{2a}$, $R^3$, $R^4$, $R^5$ and m have the same meanings as defined above and Y represents a removable group, with a compound represented by the following general formula or a salt thereof:

$H\text{-}R^{6b}$

wherein $R^{6b}$ has the same meaning as defined above.

11. Use of a 1,2-ethanediol derivative represented by the following general formula or a salt thereof for preparing a drug for treating cerebrovascular dementia, senile dementia, Alzheimer's dementia, sequelae of ischemic encephaiopathy or cerebral apoplexy in a patient:

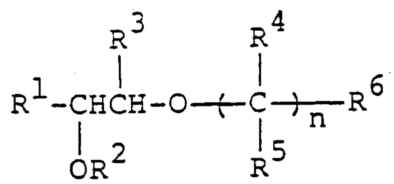

wherein $R^1$, $R^2$, $R^3$, $R^4$, $R^5$, $R^6$ and n are as defined in claim 1.

12. Use of a 1,2-ethanediol derivative or a salt thereof according to Claim 11, wherein $R^1$ represents a substituted or unsubstituted furyl, thienyl, pyridyl, quinolyl, benzothienyl, 2,3-dihydrobenzofuranyl, indolyl, benzofuranyl, 2,3-dihydrobenzothienyl, 1,2,3,4-tetrahydroquinolinyl or imidazolyl group; $R^6$ represents an ammonio group or a substituted or unsubstituted amino or nitrogen-containing heterocyclic group, the nitrogen-containing heterocyclic group being selected from the group consisting of pyrrolinyl, piperidyl, piperazinyl, imidazolyl, pyridyl, morpholinyl, thiomorpholinyl, tetrahydropyridyl, quinuclidinyl and tetrazolyl, the substituent on $R^1$ is selected from the group consisting of halogen atoms; a $C_{1-6}$ alkyl groups; $C_{2-6}$ alkenyl groups; phenyl groups which may optionally be substituted by a halogen atom or a $C_{1-6}$ alkyl or $C_{1-6}$ alkoxy group; phenyl-$C_{1-4}$alkyl groups; $C_{1-6}$ alkoxy groups which may optionally be substituted by a pyridyl, furyl or thienyl group; phenyl-$C_{1-4}$alkoxy groups which may optionally be substituted by a halogen atom or a $C_{1-6}$ alkyl, hydroxyl or $C_{1-6}$ alkoxy group; a phenoxy group; phenylaminocarbonyloxy groups which may optionally be substituted by a halogen atom; $C_{1-6}$ alkylaminocarbonyloxy groups; $C_{1-6}$ alkylthiogroups; a phenylsulfonylamino group; amino groups which may optionally be substituted by a $C_{1-6}$ alkyl group; a hydroxyl group; a nitro group; an oxo group; phenyl-$C_{1-4}$alkylthio groups; phenyl-$C_{1-4}$alkylsulfonyl groups; a thienyl group; a pyridyl group; and $C_{1-4}$ alkylenedioxy groups; the substituent on $R^6$, when $R^6$ is a substituted amino group, is selected from the group consisting of $C_{1-6}$ alkyl groups which may optionally be substituted by a hydroxyl group; $C_{1-6}$ acyl groups; cycloalkyl groups; phenyl-$C_{1-4}$-alkyl groups; a pyrimidinyl group; a pyridinecarbonyl group and an adamantyl group; and the substituent on $R^6$, when $R^6$ is a substituted

nitrogen-containing heterocyclic group, is selected from the group consisting of halogen atoms; a hydroxyl group; $C_{1-6}$ alkyl groups which may optionally be substituted by a hydroxyl group; an amino group; $C_{1-6}$ acyl groups which may optionally be substituted by a pyrrolidinyl group; phenyl-$C_{1-4}$alkyl groups; phenyl-$C_{2-4}$alkenyl groups; aroyl groups which are substituted by a halogen atom; a pyridyl group and an oxo group.

**13.** Use of a 1,2-ethanediol derivative or a salt thereof according to Claim 12, wherein $R^1$ represents a benzothienyl, benzofuranyl or 2,3-dihydrobenzothienyl group; $R^2$ represents a hydrogen atom; $R^3$ represents a hydrogen atom; $nR^4$'s and $nR^5$'s represent hydrogen atoms; $R^6$ represents a pyridyl group; a piperidinyl group which may optionally be substituted by a $C_{1-6}$ alkyl group; a 1,2,5,6-tetrahydropyridyl group which may optionally be substituted by a $C_{1-6}$ alkyl group; an imidazolyl group; a phenyl-$C_{1-4}$alkyl group-substituted piperazinyl group or an amino group which may optionally be substituted by a $C_{1-6}$ alkyl group or a cycloalkyl group; and n represents an integer of 1 to 4.

**14.** Use of a 1,2-ethanediol derivative or a salt thereof according to Claim 11, wherein the derivative or salt is 1-(benzo[b]thiophen-5-yl)-2-[2-(N,N-dimethylamino)ethoxy]ethanol or a salt thereof.

**15.** Use of a 1,2-ethanediol derivative or a salt thereof according to Claim 11, wherein the derivative or salt is 1-(benzo[b]thiophen-5-yl)-2-[3-(N,N-dimethylamino)propoxy]ethanol or a salt thereof.

**16.** Use of a 1,2-ethanediol derivative or a salt thereof according to Claim 11, wherein the derivative or salt is 1-(benzo[b]thiophen-5-yl)-2-(imidazolylmethoxy)ethanol or a salt thereof.

**17.** Use of a 1,2-ethanediol derivative or a salt thereof according to Claim 11, wherein the derivative or salt is 1-(benzo[b]thiophen-5-yl)-2-[(1-methyl-1,2,5,6-tetrahydropyridin-3-yl)methoxy]ethanol or a salt thereof.

**18.** Use of a 1,2-ethanediol derivative or a salt thereof according to Claim 11, wherein the derivative or salt is 1-(benzo[b]furan-5-yl)-2-[2-(N,N-dimethylamino)ethoxy]ethanol or a salt thereof.

**19.** Use of a 1,2-ethanediol derivative or a salt thereof according to Claim 11, wherein the derivative or salt is 1-(benzo[b]thiophen-5-yl)-2-[2-(N,N-diethylamino)ethoxy]ethanol or a salt thereof.

**20.** A cerebral function-improving agent comprising a 1,2-ethanediol derivative represented by the following general formula or a salt thereof:

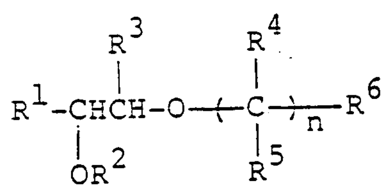

wherein $R^1$, $R^2$, $R^3$, $R^4$, $R^5$, $R^6$, and n are defined in claim 1.

**Patentansprüche**

**1.** Ein 1,2-Ethandiolderivat der folgenden allgemeinen Formel oder dessen Salz:

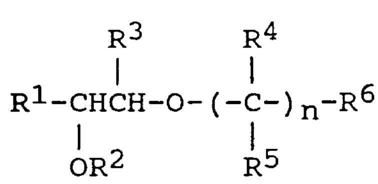

worin $R^1$ eine substituierte oder unsubstituierte heterocyclische Gruppe, $R^2$ ein Wasserstoffatom, eine

$C_1$-$C_6$-Alkylgruppeoder eine Hydroxyl-Schutzgruppe, $R^3$ ein Wasserstoffatom oder eine $C_1$-$C_6$-Alkylgruppe bedeutet, die Gruppen $nR^4$ und $nR^5$ untereinander gleich oder verschieden sind und Wasserstoffatome oder $C_1$-$C_6$-Alkylgruppen bedeuten, $R^6$ eine Ammoniumgruppe oder eine substituierte oder unsubstituierte Amino- oder stickstoffhaltige heterocyclische Gruppe bedeutet, wobei die stickstoffhaltige heterocyclische Gruppe aus der aus Pyrrolyl-, Pyrrolidinyl-, Piperidyl-, Piperazinyl-, Imidazolyl-, Pyrazolyl-, Pyridyl-, Tetrahydropyridyl-, Pyrimidinyl-, Morpholinyl-, Thiomorpholinyl-, Chinolyl-, Chinolizinyl-, Tetrahydrochinolinyl-, Tetrahydroisochinolinyl-, Chinuclidinyl-, Thiazolyl-, Tetrazolyl-, Thiadiazolyl-, Pyrrolinyl-, Imidazolinyl-, Imidazolidinyl-, Pyrazolinyl-, Pyrazolidinyl-, Purinyl- und Indazolylgruppen bestehenden Gruppe ausgewählt wird; n 0 oder eine ganze Zahl von 1 bis 6 bedeutet, wobei der Substituent an $R^1$ aus der aus Halogenatomen, substituierten oder unsubstituierten Amino-, $C_1$-$C_6$-Alkyl-, Aryl-, Ar-$C_1$-$C_4$-Alkyl-, $C_1$-$C_6$-Alkoxy-, Ar-$C_1$-$C_4$-Alkoxy-, Aryloxy-, Carbamoyloxy-, $C_1$-$C_6$-Alkylthio-, $C_2$-$C_6$-Alkenyl-, $C_2$-$C_6$-Alkenyloxy-, Ar-$C_1$-$C_4$-Alkylthio-, Ar-$C_1$-$C_6$-Alkylsulfonyl-, Arylsulfonyl-, $C_1$-$C_6$-Alkylsulfonylamino-, Arylsulfonylamino- und heterocyclischen Gruppen, geschützten Aminogruppen, geschützten oder ungeschützten Hydroxylgruppen, der Nitrogruppe, der Oxogruppe und $C_1$-$C_4$-Alkylendioxygruppen bestehenden Gruppe ausgewählt wird; wobei die substituierte $C_1$-$C_6$-Alkyl-, Aryl-, Ar-$C_1$-$C_4$-Alkyl-, $C_1$-$C_6$-Alkoxy-, Ar-$C_1$-$C_4$-Alkoxy-, Aryloxy-, Carbamoyloxy-, $C_1$-$C_6$-Alkylthio-,$C_2$-$C_6$-Alkenyl-, $C_2$-$C_6$-Alkenyloxy-, Ar-$C_1$-$C_4$-Alkylthio-, Ar-$C_1$-$C_6$-Alkylsulfonyl-, Arylsulfonyl-, $C_1$-$C_6$-Alkylsulfonylamino-, Arylsulfonylamino- oder heterocyclische Gruppe als Substituent von $R^1$ und die substituierte stickstoffhaltige heterocyclische Gruppe als $R^6$ jeweils mindestens einen aus der aus Halogenatomen, geschützten oder ungeschützten Hydroxylgruppen, geschützten oder ungeschützten Aminogruppen, geschützten oder ungeschützten Carboxylgruppen, unsubstituierten $C_1$-$C_6$-Alkylgruppen, durch eine geschützte oder ungeschützte Hydroxylgruppe substituierten $C_1$-$C_6$-Alkylgruppen, unsubstituierten oder halogensubstituierten Arylgruppen, unsubstituierten oder halogensubstituierten Aroylgruppen, unsubstituierten $C_1$-$C_6$-Alkoxygruppen, durch eine $C_1$-$C_6$-Alkoxygruppe substituierten $C_1$-$C_6$-Alkoxygruppen, $C_1$-$C_6$-Acylgruppen, Ar-$C_1$-$C_4$-Alkylgruppen, Ar-$C_2$-$C_6$-Alkenylgruppen, heterocyclischen Gruppen, heterocyclischen CO-Gruppen, der Oxogruppe, $C_1$-$C_6$-Alkylsulfonylgruppen und Arylsulfonylgruppen bestehenden Gruppe ausgewählten Substituenten aufweist; und die substituierte Aminogruppe als Substituent von $R^1$ und die substituierte Aminogruppe als $R^6$ jeweils mindestens einen aus der aus geschützten oder ungeschützten Hydroxylgruppen, unsubstituierten $C_1$-$C_6$-Alkylgruppen, durch eine geschützte oder ungeschützte Carboxyl- oder Hydroxylgruppe substituierten $C_1$-$C_6$-Alkylgruppen, Cycloalkylgruppen, Arylgruppen, $C_1$-$C_6$-Acylgruppen, Ar-$C_1$-$C_4$-Alkylgruppen, heterocyclischen Gruppen, unsubstituierten oder Oxo-substituierten heterocyclischen CO-Gruppen, der Adamantylgruppe, $C_1$-$C_6$-Alkylsulfonylgruppen und Arylsulfonylgruppen bestehenden Gruppe ausgewählten Substituenten tragen, wobei die obigen heterocyclischen Gruppen aus der aus stickstoffhaltigen heterocyclischen Gruppen der für $R^6$ genannten Bedeutung und Furyl-, Thienyl-, Benzothienyl-, Pyranyl-, Isobenzofuranyl-, Oxazolyl-, Benzofuranyl-, Indolyl-, Benzimidazolyl-, Benzoxazolyl-, Benzothiazolyl-, Chinoxalyl-, Dihydrochinoxalyl-, 2,3-Dihydrobenzothienyl-, 2,3-Dihydrobenzopyrrolyl-, 2,3-Dihydro-4H-1-thianaphthyl-, 2,3-Dihydrobenzofuranyl-, Benzo[b]dioxanyl-, Imidazo[2,3-a]pyridyl-, Benzo[b]piperazinyl-, Chromenyl-, Isothiazolyl-, Isoxazolyl-, Oxadiazolyl-, Pyridazinyl-, Isoindolyl- und Isochinolylgruppen bestehenden Gruppe ausgewählt werden.

**2.** Ein 1,2-Ethandiolderivat oder dessen Salz gemäß Anspruch 1, worin $R^1$ eine Benzothienyl-, Benzofuranyl- oder 2,3-Dihydrobenzothienylgruppe, $R^2$ ein Wasserstoffatom, $R^3$ ein Wasserstoffatom, die Gruppen $nR^4$ und $nR^5$ Wasserstoffatome und $R^6$ eine Pyridylgruppe, eine gegebenenfalls durch eine $C_1$-$C_6$-Alkylgruppe substituierte Piperidinylgruppe, eine gegebenenfalls durch eine $C_1$-$C_6$-Alkylgruppe substituierte 1,2,5,6-Tetrahydropyridylgruppe, eine Imidazolylgruppe, eine durch eine Phenyl-$C_1$-$C_4$-alkylgruppe substituierte Piperazinylgruppe oder eine gegebenenfalls durch eine $C_1$-$C_6$-Alkyl- oder Cycloalkylgruppe substituierte Aminogruppe und n eine ganze Zahl von 1 bis 4 bedeutet.

**3.** 1-(Benzo[b]thiophen-5-yl)-2-[2-(N,N-dimethyamino)ethoxy]ethanoloder dessen Salz.

**4.** 1-(Benzo[b]thiophen-5-yl)-2-[3-(N,N-dimethylamino)propoxy]ethanol oder dessen Salz.

**5.** 1-(Benzo[b]thiophen-5-yl)-2-(imidazolylmethoxy)ethanol oder dessen Salz.

**6.** 1-(Benzo[b]thiophen-5-yl)-2-[(1-methyl-1,2,5,6-tetrahydropyridin-3-yl)methoxy]ethanol oder dessen Salz.

**7.** 1-(Benzo[b]furan-5-yl)-2-[2-(N,N-dimethylamino)ethoxy]ethanol oder dessen Salz.

**8.** 1-(Benzo[b]thiophen-5-yl)-2-[2-(N,N-diethylamino)ethoxy]ethanoloder dessen Salz.

**9.** Ein Verfahren zur Herstellung eines 1,2-Ethandiolderivats der folgenden allgemeinen Formel oder dessen Salzes:

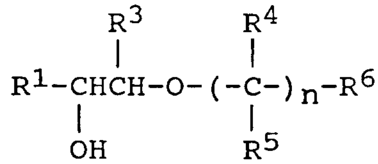

worin $R^1$ eine substituierte oder unsubstituierte heterocyclische Gruppe, $R^3$ ein Wasserstoffatom oder eine $C_1$-$C_6$-Alkylgruppe bedeutet, die Gruppen $nR^4$ und $nR^5$ untereinander gleich oder verschieden sind und Wasserstoffatome oder $C_1$-$C_6$-Alkylgruppen bedeuten, $R^6$ eine Ammoniumgruppe oder eine substituierte oder unsubstituierte Amino- oder stickstoffhaltige heterocyclische Gruppe bedeutet, wobei die stickstoffhaltige heterocyclische Gruppe aus der aus Pyrrolyl-, Pyrrolidinyl-, Piperidyl-, Piperazinyl-, Imidazolyl-, Pyrazolyl-, Pyridyl-, Tetrahydropyridyl-, Pyrimidinyl-, Morpholinyl-, Thiomorpholinyl-, Chinolyl-, Chinolizinyl-, Tetrahydrochinolinyl-, Tetrahydroisochinolinyl-, Chinuclidinyl-, Thiazolyl-, Tetrazolyl-, Thiadiazolyl-, Pyrrolinyl-, Imidazolinyl-, Imidazolidinyl-, Pyrazolinyl-, Pyrazolidinyl-, Purinyl- und Indazolylgruppen bestehenden Gruppe ausgewählt wird; n 0 oder eine ganze Zahl von 1 bis 6 bedeutet, wobei der Substituent an $R^1$ aus der aus Halogenatomen, substituierten oder unsubstituierten Amino-, $C_1$-$C_6$-Alkyl-, Aryl-, Ar-$C_1$-$C_4$-Alkyl-, $C_1$-$C_6$-Alkoxy-, Ar-$C_1$-$C_4$-Alkoxy-, Aryloxy-, Carbamoyloxy-, $C_1$-$C_6$-Alkylthio-, $C_2$-$C_6$-Alkenyl-, $C_2$-$C_6$-Alkenyloxy-, Ar-$C_1$-$C_4$-Alkylthio-, Ar-$C_1$-$C_6$-Alkylsulfonyl-, Arylsulfonyl-, $C_1$-$C_6$-Alkylsulfonylamino-, Arylsulfonylamino- und heterocyclischen Gruppen, geschützten Aminogruppen, geschützten oder ungeschützten Hydroxylgruppen, der Nitrogruppe, der Oxogruppe und $C_1$-$C_4$-Alkylendioxygruppen bestehenden Gruppe ausgewählt wird; wobei die substituierte $C_1$-$C_6$-Alkyl-, Aryl-, Ar-$C_1$-$C_4$-Alkyl-, $C_1$-$C_6$-Alkoxy-, Ar-$C_1$-$C_4$-Alkoxy-, Aryloxy-, Carbamoyloxy-, $C_1$-$C_6$-Alkylthio-, $C_2$-$C_6$-Alkenyl-, $C_2$-$C_6$-Alkenyloxy-, Ar-$C_1$-$C_4$-Alkylthio-, Ar-$C_1$-$C_6$-Alkylsulfonyl-, Arylsulfonyl-, $C_1$-$C_6$-Alkylsulfonylamino-, Arylsulfonylamino- oder heterocyclische Gruppe als Substituent von $R^1$ und die substituierte stickstoffhaltige heterocyclische Gruppe als $R^6$ jeweils mindestens einen aus der aus Halogenatomen, geschützten oder ungeschützten Hydroxylgruppen, geschützten oder ungeschützten Aminogruppen, geschützten oder ungeschützten Carboxylgruppen, unsubstituierten $C_1$-$C_6$-Alkylgruppen, durch eine geschützte oder ungeschützte Hydroxylgruppe substituierten $C_1$-$C_6$-Alkylgruppen, unsubstituierten oder halogensubstituierten Arylgruppen, unsubstituierten oder halogen-substituierten Aroylgruppen, unsubstituierten $C_1$-$C_6$-Alkoxygruppen, durch eine $C_1$-$C_6$-Alkoxygruppe substituierten $C_1$-$c_6$-Alkoxygruppen, $C_1$-$C_6$-Acylgruppen, Ar-$C_1$-$C_4$-Alkylgruppen, Ar-$C_2$-$C_6$-Alkenylgruppen, heterocyclischen Gruppen, heterocyclischen CO-Gruppen, der Oxogruppe, $C_1$-$C_6$-Alkylsulfonylgruppen und Arylsulfonylgruppen bestehenden Gruppe ausgewählten Substituenten aufweist; und die substituierte Aminogruppe als Substituent von $R^1$ und die substituierte Aminogruppe als $R^6$ jeweils mindestens einen aus der aus geschützten oder ungeschützten Hydroxylgruppen, unsubstituierten $C_1$-$C_6$-Alkylgruppen, durch eine geschützte oder ungeschützte Carboxyl- oder Hydroxylgruppe substituierten $C_1$-$C_6$-Alkylgruppen, Cycloalkylgruppen, Arylgruppen, $C_1$-$C_6$-Acylgruppen, Ar-$C_1$-$C_4$-Alkylgruppen, heterocyclischen Gruppen, unsubstituierten oder Oxo-substituierten heterocyclischen CO-Gruppen, der Adamantylgruppe, $C_1$-$C_6$-Alkylsulfonylgruppen und Arylsulfonylgruppen bestehenden Gruppe ausgewählten Substituenten aufweist, wobei die obigen heterocyclischen Gruppen aus der aus stickstoffhaltigen heterocyclischen Gruppen der für $R^6$ genannten Bedeutung und Furyl-, Thienyl-, Benzothienyl-, Pyranyl-, Isobenzofuranyl-, Oxazolyl-, Benzofuranyl-, Indolyl-, Benzimidazolyl-, Benzoxazolyl-, Benzothiazolyl-, Chinoxalyl-, Dihydrochinoxalyl, 2,3-Dihydrobenzothienyl-, 2,3-Dihydrobenzopyrrolyl-, 2,3-Dihydro-4H-1-thianaphthyl-, 2,3-Dihydrobenzofuranyl-, Benzo[b]dioxanyl-, Imidazo[2,3-a]pyridyl-, Benzo[b]piperazinyl-, Chromenyl-, Isothiazolyl-, Isoxazolyl-, Oxadiazolyl-, Pyridazinyl-, Isoindolyl- und Isochinolylgruppen bestehenden Gruppe ausgewählt werden, welches die Umsetzung einer Verbindung der allgemeinen Formel:

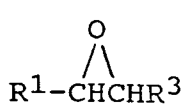

worin $R^1$ und $R^3$ die obengenannten Bedeutungen haben, mit einer Verbindung der folgenden allgemeinen Formel oder deren Salz:

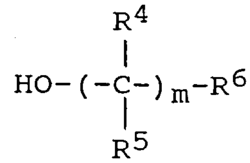

worin die Reste $mR^4$ und $mR^5$ untereinander gleich oder verschieden sind und Wasserstoffatome oder Niedrigalkylgruppen bedeuten, $R^6$ die obengenannte Bedeutung hat und m eine ganze Zahl von 1 bis 6 bedeutet, oder einer Verbindung der folgenden allgemeinen Formel oder deren Salz:

$HO-R^{6a}$

worin $R^{6a}$ die gleiche substituierte oder unsubstituierte stickstoffhaltige heterocyclische Gruppe wie für $R^6$ definiert bedeutet, unter der Voraussetzung, daß die heterocyclische Gruppe an einem den heterocyclischen Ring bildenden Kohlenstoffatom eine freie Valenz aufweist, umfaßt.

**10.** Ein Verfahren zur Herstellung eines 1,2-Ethandiolderivats der folgenden allgemeinen Formel oder dessen Salzes:

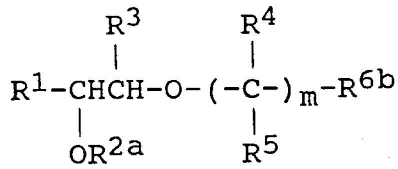

worin $R^1$ eine substituierte oder unsubstituierte heterocyclische Gruppe, $R^{2a}$ eine Hydroxyl-Schutzgruppe, $R^3$ ein Wasserstoffatom oder eine $C_1$-$C_6$-Alkylgruppe bedeutet, die Gruppen $mR^4$ und $mR^5$ untereinander gleich oder verschieden sind und Wasserstoffatome oder $C_1$-$C_6$-Alkylgruppen bedeuten, $R^{6b}$ eine substituierte oder unsubstituierte Amino- oder stickstoffhaltige heterocyclische Gruppe bedeutet, wobei die stickstoffhaltige heterocyclische Gruppe eine freie Valenz an einem den heterocyclischen Ring bildenden Stickstoffatom aufweist und aus der aus Pyrrolyl-, Pyrrolidinyl-, Piperidyl-, Piperazinyl-, Imidazolyl-, Pyrazolyl-, Pyridyl-, Tetrahydropyridyl-, Pyrimidinyl-, Morpholinyl-, Thiomorpholinyl-, Chinolyl-, Chinolizinyl-, Tetrahydrochinolinyl-, Tetrahydroisochinolinyl-, Chinuclidinyl-, Thiazolyl-, Tetrazolyl-, Thiadiazolyl-, Pyrrolinyl-, Imidazolinyl-, Imidazolidinyl-, Pyrazolinyl-, Pyrazolidinyl-, Purinyl- und Indazolylgruppen bestehenden Gruppe ausgewählt wird; m eine ganze Zahl von 1 bis 6 bedeutet, wobei der Substituent an $R^1$ aus der aus Halogenatomen, substituierten oder unsubstituierten Amino-, $C_1$-$C_6$-Alkyl-, Aryl-, Ar-$C_1$-$C_4$-Alkyl-, $C_1$-$C_6$-Alkoxy-, Ar-$C_1$-$C_4$-Alkoxy-, Aryloxy-, Carbamoyloxy-, $C_1$-$C_6$-Alkylthio-, $C_2$-$C_6$-Alkenyl-, $C_2$-$C_6$-Alkenyloxy-, Ar-$C_1$-$C_4$-Alkylthio-, Ar-$C_1$-$C_6$-Alkylsulfonyl-, Arylsulfonyl-, $C_1$-$C_6$-Alkylsulfonylamino-, Arylsulfonylamino- und heterocyclischen Gruppen, geschützten Aminogruppen, geschützten oder ungeschützten Hydroxylgruppen, der Nitrogruppe, der Oxogruppe und $C_1$-$C_4$-Alkylendioxygruppen bestehenden Gruppe ausgewählt wird; wobei die substituierte $C_1$-$C_6$-Alkyl-, Aryl-, Ar-$C_1$-$C_4$-Alkyl-, $C_1$-$C_6$-Alkoxy-, Ar-$C_1$-$C_4$-Alkoxy-, Aryloxy-, Carbamoyloxy-, $C_1$-$C_6$-Alkylthio-, $C_2$-$C_6$-Alkenyl-, $C_2$-$C_6$-Alkenyloxy-, Ar-$C_1$-$C_4$-Alkylthio-, Ar-$C_1$-$C_6$-Alkylsulfonyl-, Arylsulfonyl-, $C_1$-$C_6$-Alkylsulfonylamino-, Arylsulfonylamino- oder heterocyclische Gruppe als Substituent von $R^1$ und die substituierte stickstoffhaltige heterocyclische Gruppe als $R^{6b}$ jeweilsmindestens einen aus der aus Halogenatomen, geschützten oder ungeschützten Hydroxylgruppen, geschützten oder ungeschützten Aminogruppen, geschützten oder ungeschützten Carboxylgruppen, unsubstituierten $C_1$-$C_6$-Alkylgruppen, durch eine geschützte oder ungeschützte Hydroxylgruppe substituierten $C_1$-$C_6$-Alkylgruppen, unsubstituierten oder halogensubstituierten Arylgruppen, unsubstituierten oder halogensubstituierten Aroylgruppen, unsubstituierten $C_1$-$C_6$-Alkoxygruppen, durch eine $C_1$-$C_6$-Alkoxygruppe substituierten $C_1$-$C_6$-Alkoxygruppen, $C_1$-$C_6$-Acylgruppen, Ar-$C_1$-$C_4$-Alkylgruppen, Ar-$C_2$-$C_6$-Alkenylgruppen, heterocyclischen Gruppen, heterocyclischen CO-Gruppen, der Oxogruppe, $C_1$-$C_6$-Alkylsulfonylgruppen und Arylsulfonylgruppen bestehenden

Gruppe ausgewählten Substituenten aufweist; und die substituierte Aminogruppe als Substituent von $R^1$ und die substituierte Aminogruppe als $R^{6b}$ jeweils mindestens einen aus der aus geschützten oder ungeschützten Hydroxylgruppen, unsubstituierten $C_1$-$C_6$-Alkylgruppen, durch eine geschützte oder ungeschützte Carboxyl- oder Hydroxylgruppe substituierten $C_1$-$C_6$-Alkylgruppen, Cycloalkylgruppen, Arylgruppen, $C_1$-$C_6$-Acylgruppen, Ar-$C_1$-$C_4$-Alkylgruppen, heterocyclischen Gruppen, unsubstituierten oder Oxo-substituierten heterocyclischen CO-Gruppen, der Adamantylgruppe, $C_1$-$C_6$-Alkylsulfonylgruppen und Arylsulfonylgruppen bestehenden Gruppe ausgewählten Substituenten aufweist, wobei die obigen heterocyclischen Gruppen aus der aus stickstoffhaltigen heterocyclischen Gruppen der für $R^{6b}$ genannten Bedeutung und Furyl-, Thienyl-, Benzothienyl-, Pyranyl-, Isobenzofuranyl-, Oxazolyl-, Benzofuranyl-, Indolyl-, Benzimidazolyl, Benzoxazolyl-, Benzothiazolyl-, Chinoxalyl-, Dihydrochinoxalyl-, 2,3-Dihydrobenzothienyl-, 2,3-Dihydrobenzopyrrolyl-, 2,3-Dihydro-4H-1-thianaphthyl-, 2,3-Dihydrobenzofuranyl-, Benzo[b]dioxanyl-, Imidazo[2,3-a]pyridyl-, Benzo[b]piperazinyl-, Chromenyl-, Isothiazolyl-, Isoxazolyl-, Oxadiazolyl-, Pyridazinyl-, Isoindolyl- und Isochinolylgruppen bestehenden Gruppe ausgewählt werden, welches die Umsetzung einer Verbindung der allgemeinen Formel:

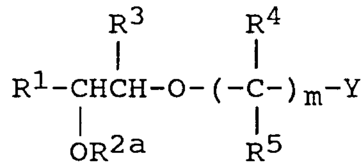

worin $R^1$, $R^{2a}$, $R^3$, $R^4$, $R^5$ und m die obengenannte Bedeutung haben und Y eine abspaltbare Gruppe bedeutet, mit einer Verbindung der folgenden allgemeinen Formel oder deren Salz:

$H$-$R^{6b}$

worin $R^{6b}$ die obengenannte Bedeutung hat, umfaßt.

**11.** Verwendung eines 1,2-Ethandiolderivats der folgenden allgemeinen Formel oder dessen Salzes zur Herstellung eines Arzneimittels für die Behandlung zerebrovaskulärer Demenz, seniler Demenz, Alzheimerscher Demenz und der Folgen von ischämischer Enzephalopathie oder des zerebralen Apoplexes am Patienten:

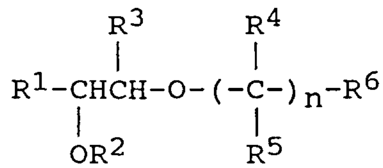

worin $R^1$, $R^2$, $R^3$, $R^4$, $R^5$, $R^6$ und n wie in Anspruch 1 definiert sind.

**12.** Verwendung eines 1,2-Ethandiolderivats oder dessen Salzes gemäß Anspruch 11, worin $R^1$ eine substituierte oder unsubstituierte Furyl-, Thienyl-, Pyridyl-, Chinolyl-, Benzothienyl-, 2,3-Dihydrobenzofuranyl-, Indolyl-, Benzofuranyl-, 2,3-Dihydrobenzothienyl-,1,2,3,4-Tetrahydrochinolinyl- oder Imidazolylgruppe bedeutet, $R^6$ eine Ammoniumgruppe oder eine substituierte oder unsubstituierte Amino- oder stickstoffhaltige heterocyclische Gruppe bedeutet, wobei die stickstoffhaltige heterocyclische Gruppe aus der aus Pyrrolinyl-, Piperidyl, Piperazinyl-, Imidazolyl-, Pyridyl-, Morpholinyl-, Thiomorpholinyl-, Tetrahydropyridyl-, Chinuclidinyl- und Tetrazolylgruppen bestehenden Gruppe ausgewählt wird, wobei der Substituent an $R^1$ aus der aus Halogenatomen, $C_1$-$C_6$-Alkylgruppen, $C_2$-$C_6$-Alkenylgruppen, gegebenenfalls durch ein Halogenatom oder eine $C_1$-$C_6$-Alkylgruppe oder eine $C_1$-$C_6$-Alkoxygruppe substituierten Phenylgruppen, Phenyl-$C_1$-$C_4$-Alkylgruppen, gegebenenfalls durch eine Pyridyl-, Furyl- oder Thienylgruppe substituierten $C_1$-$C_6$-Alkoxygruppen, gegebenenfalls durch ein Halogenatom oder eine $C_1$-$C_6$-Alkyl-, Hydroxyl- oder $C_1$-$C_6$-Alkoxygruppe substituierten Phenyl-$C_1$-$C_4$-Alkoxygruppen, einer Phenoxygruppe, gegebenenfalls durch ein Halogenatom substituierten Phenylaminocarbonyloxygruppen, $C_1$-$C_6$-Alkylaminocarbonyloxygruppen, $C_1$-$C_6$-Alkylthiogruppen, einer Phenylsulfonylaminogruppe,

gegebenenfalls durch eine $C_1$-$C_6$-Alkylgruppe substituierten Aminogruppen, einer Hydroxylgruppe, einer Nitrogruppe, einer Oxogruppe, Phenyl-$C_1$-$C_4$-alkylthiogruppen, Phenyl-$C_1$-$C_4$-alkylsulfonylgruppen, einer Thienylgruppe, einer Pyridylgruppe und $C_1$-$C_4$-Alkylendioxygruppen bestehenden Gruppe ausgewählt wird, wobei der Substituent an $R^6$, wenn $R^6$ eine substituierte Aminogruppe ist, aus der aus gegebenenfalls durch eine Hydroxylgruppe substituierten $C_1$-$C_6$-Alkylgruppen, $C_1$-$C_6$-Acylgruppen, Cycloalkylgruppen, Phenyl-$C_1$-$C_4$-alkylgruppen, einer Pyrimidinylgruppe, einer Pyridincarbonylgruppe und einer Adamantylgruppe bestehenden Gruppe ausgewählt wird, und der Substituent an $R^6$, wenn $R^6$ eine substituierte stickstoffhaltige heterocyclische Gruppe ist, aus der aus Halogenatomen, einer Hydroxylgruppe, gegebenenfalls durch eine Hydroxylgruppe substituierten $C_1$-$C_6$-Alkylgruppen, einer Aminogruppe, gegebenenfalls durch eine Pyrrolidinylgruppe substituierten $C_1$-$C_6$-Acylgruppen, Phenyl-$C_1$-$C_4$-alkylgruppen, Phenyl-$C_2$-$C_4$-alkenylgruppen, durch ein Halogenatom substituierten Aroylgruppen, einer Pyridylgruppe und einer Oxogruppe bestehenden Gruppe ausgewählt wird.

**13.** Verwendung eines 1,2-Ethandiolderivats oder dessen Salzes gemäß Anspruch 12, worin $R^1$ eine Benzothienyl-, Benzofuranyl- oder 2,3-Dihydrobenzothienylgruppe bedeutet, $R^2$ ein Wasserstoffatom bedeutet, $R^3$ ein Wasserstoffatom bedeutet, die Gruppen $nR^4$ und $nR^5$ Wasserstoffatome bedeuten, $R^6$ eine Pyridylgruppe, eine gegebenfalls durch eine $C_1$-$C_6$-Alkylgruppe substituierte Piperidinylgruppe, eine gegebenfalls durch eine $C_1$-$C_6$-Alkylgruppe substituierte 1,2,5,6-Tetrahydropyridylgruppe, eine Imidazolylgruppe, eine durch eine Phenyl-$C_1$-$C_4$-alkylgruppe substituierte Piperazinylgruppe oder eine gegebenfalls durch eine $C_1$-$C_6$-Alkylgruppe oder eine Cycloalkylgruppe substituierte Aminogruppe bedeutet und n eine ganze Zahl von 1 bis 4 bedeutet.

**14.** Verwendung eines 1,2-Ethandiolderivats oder dessen Salzes gemäß Anspruch 11, worin das Derivat oder Salz 1-(Benzo[b]-thiophen-5-yl)-2-[2-(N,N-dimethylamino)ethoxy]ethanol oder dessen Salz ist.

**15.** Verwendung eines 1,2-Ethandiolderivats oder dessen Salzes gemäß Anspruch 11, worin das Derivat oder Salz 1-(Benzo[b]-thiophen-5-yl)-2-[3-(N,N-dimethylamino)propoxy]ethanol oder dessen Salz.

**16.** Verwendung eines 1,2-Ethandiolderivats oder dessen Salzes gemäß Anspruch 11, worin das Derivat oder Salz 1-(Benzo[b]-thiophen-5-yl)-2-(imidazolylmethoxy)ethanol oder dessen Salz ist.

**17.** Verwendung eines 1,2-Ethandiolderivats oder dessen Salzes gemäß Anspruch 11, worin das Derivat oder Salz 1-(Benzo[b]-thiophen-5-yl)-2-[(1-methyl-1,2,5,6-tetrahydropyridin-3-yl)methoxy]ethanol oder dessen Salz ist.

**18.** Verwendung eines 1,2-Ethandiolderivats oder dessen Salzes gemäß Anspruch 11, worin das Derivat oder Salz 1-(Benzo[b]-furan-5-yl)-2-[2-(N,N-dimethylamino)ethoxy]ethanol oder dessen Salz ist.

**19.** Verwendung eines 1,2-Ethandiolderivats oder dessen Salzes gemäß Anspruch 11, worin das Derivat oder Salz 1-(Benzo[b]-thiophen-5-yl)-2-[2-(N,N-diethylamino)ethoxy]ethanol oder dessen Salz ist.

**20.** Ein gehirnfunktionsverbesserndes Mittel, das ein 1,2-Ethandiolderivat der folgenden allgemeinen Formel oder dessen Salz enthält:

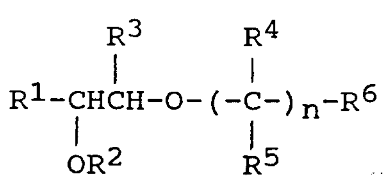

worin $R^1$, $R^2$, $R^3$, $R^4$, $R^5$, $R^6$ und n wie in Anspruch 1 definiert sind.

## Revendications

**1.** Dérivé de 1,2-éthanediol, représenté par la formule générale suivante, ou un sel de ce dérivé :

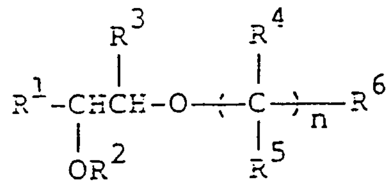

formule dans laquelle :

- $R^1$ représente un groupe hétérocyclique substitué ou non substitué ;
- $R^2$ représente un atome d'hydrogène, un groupe alkyle en $C_{1-6}$ ou un groupe protecteur d'hydroxyle ;
- $R^3$ représente un atome d'hydrogène ou un groupe alkyle en $C_{1-6}$ ;
- les $nR^4$ et les $nR^5$ sont identiques ou différents les uns des autres et représentent des atomes d'hydrogène ou des groupes alkyle en $C_{1-6}$ ;
- $R^6$ représente un groupe ammonio ou un groupe amino ou hétérocyclique contenant de l'azote, substitué ou non substitué, le groupe hétérocyclique contenant de l'azote étant choisi dans le groupe constitué par les groupes pyrrolyle, pyrrolidinyle, pipéridyle, pipérazinyle, imidazolyle, pyrazolyle, pyridyle, tétrahydropyridyle, pyrimidinyle, morpholinyle, thiomorpholinyle, quinolyle, quinolizinyle, tétrahydroquinolinyle, tétrahydroisoquinolinyle, quinuclidinyle, thiazolyle, tétrazolyle, thiadiazolyle, pyrrolinyle, imidazolinyle, imidazolidinyle, pyrazolinyle, pyrazolidinyle, purinyle et indazolyle ; et
- n représente 0 ou un nombre entier de 1 à 6,

le substituant sur $R^1$ étant choisi dans le groupe constitué par les atomes d'halogène, les groupes amino, alkyle en $C_{1-6}$, aryle, ar-alkyle en $C_{1-4}$, alcoxy en $C_{1-6}$, ar-alcoxy en $C_{1-4}$, aryloxy, carbamoyloxy, alkylthio en $C_{1-6}$, alcényle en $C_{2-6}$, alcényloxy en $C_{2-6}$, ar-alkylthio en $C_{1-4}$, ar-alkylsulfonyle en $C_{1-6}$, arylsulfonyle, alkylsulfonylamino en $C_{1-6}$, arylsulfonylamino et hétérocycliques, substitués ou non substitués, les groupes amino protégés, les groupes hydroxyle protégés ou non protégés, le groupe nitro, le groupe oxo et les groupes alkylènedioxy en $C_{1-4}$ ;
le groupe alkyle en $C_{1-6}$, aryle, ar-alkyle en $C_{1-4}$, alcoxy en $C_{1-6}$, ar-alcoxy en $C_{1-4}$, aryloxy, carbamoyloxy, alkylthio en $C_{1-6}$, alcényle en $C_{2-6}$, alcényloxy en $C_{2-6}$, ar-alkylthio en $C_{1-4}$, ar-alkylsulfonyle en $C_{1-6}$, arylsulfonyle, alkylsulfonylamino en $C_{1-6}$, arylsulfonylamino ou hétérocyclique, substitué, en tant que substituant de $R^1$, et le groupe hétérocyclique contenant de l'azote, substitué, en tant que $R^6$, ayant chacun au moins un substituant choisi dans le groupe constitué par les atomes d'halogène, les groupes hydroxyle protégés ou non protégés, les groupes amino protégés ou non protégés, les groupes carboxyle protégés ou non protégés, les groupes alkyle en $C_{1-6}$ non substitués, les groupes alkyle en $C_{1-6}$ substitués par un groupe hydroxyle protégé ou non protégé, les groupes aryle non substitués ou substitués par halogène, les groupes aroyle non substitués ou substitués par halogène, les groupes alcoxy en $C_{1-6}$ non substitués, les groupes alcoxy en $C_{1-6}$ substitués par un groupe alcoxy en $C_{1-6}$, les groupes acyle en $C_{1-6}$, les groupes ar-alkyle en $C_{1-4}$, les groupes ar-alcényle en $C_{2-6}$, les groupes hétérocycliques, les groupes hétérocyclique-CO-, le groupe oxo, les groupes alkylsulfonyle en $C_{1-6}$ et les groupes arylsulfonyle ; et
le groupe amino substitué, en tant que substituant de $R^1$, et le groupe amino substitué, en tant que $R^6$, ayant chacun au moins un substituant choisi dans le groupe constitué par les groupes hydroxyle protégés ou non protégés, les groupes alkyle en $C_{1-6}$ non substitués, les groupes alkyle en $C_{1-6}$ substitués par un groupe carboxyle ou hydroxyle, protégé ou non protégé, les groupes cycloalkyle, les groupes aryle, les groupes acyle en $C_{1-6}$, les groupes ar-alkyle en $C_{1-4}$, les groupes hétérocycliques, les groupes hétérocyclique-CO- non substitués ou substitués par oxo, le groupe adamantyle, les groupes alkylsulfonyle en $C_{1-6}$ et les groupes arylsulfonyle; tous les groupes héterocycliques ci-dessus étant choisis dans le groupe constitué par les groupes hétérocycliques contenant de l'azote mentionnés dans la définition de $R^6$ et les groupes furyle, thiényle, benzothiényle, pyrannyle, isobenzofurannyle, oxazolyle, benzofurannyle, indolyle, benzimidazolyle, benzoxazolyle, benzothiazolyle, quinoxalyle, dihydroquinoxalyle, 2,3-dihydrobenzothiényle, 2,3-dihydrobenzopyrrolyle, 2,3-dihydro-4H-1-thianaphtyle, 2,3-dihydrobenzofurannyle, benzo[b]dioxannyle, imidazo [2,3-a]pyridyle, benzo[b]pipérazinyle, chroményle, isothiazolyle, isoxazolyle, oxadiazolyle, pyridazinyle, isoindolyle et isoquinolyle.

**2.** Dérivé de 1,2-éthanediol ou sel de ce dérivé, selon la revendication 1, dans lequel :
- $R^1$ représente un groupe benzothiényle, benzofurannyle ou 2,3-dihydrobenzothiényle ;
- $R^2$ représente un atome d'hydrogène ;
- $R^3$ représente un atome d'hydrogène ;
- les $nR^4$ et les $nR^5$ représentent des atomes d'hydrogène;
- $R^6$ représente un groupe pyridyle, un groupe pipéridinyle qui peut facultativement être substitué par un groupe alkyle en $C_{1-6}$, un groupe 1,2,5,6-tétrahydropyridyle qui peut facultativement être substitué par un groupe alkyle en $C_{1-6}$, un groupe imidazolyle, un groupe pipérazinyle substitué par un groupe phényl-alkyle en $C_{1-4}$ ou un groupe amino qui peut facultativement être substitué par un groupe alkyle en $C_{1-6}$ ou cycloalkyle ; et
- n représente un nombre entier de 1 à 4.

**3.** 1-(Benzo[b]thiophène-5-yl)-2-[2-(N,N-diméthylamino)éthoxy]éthanol ou un sel de ce dérivé.

**4.** 1-(Benzo[b]thiophène-5-yl)-2-[3-(N,N-diméthylamino)propoxy]éthanol ou un sel de ce dérivé.

**5.** 1-(Benzo[b]thiophène-5-yl)-2-(imidazolylméthoxy)éthanol ou un sel de ce dérivé.

**6.** 1-(Benzo[b]thiophène-5-yl)-2-[1-méthyl-1,2,5,6-tétrahydropyridine-3-yl)méthoxy]éthanol ou un sel de ce dérivé.

**7.** 1-(Benzo[b]furanne-5-yl)-2-[2-(N,N-diméthylamino)-éthoxy]éthanol ou un sel de ce dérivé.

**8.** 1-(Benzo[b]thiophène-5-yl)-2-[2-(N,N-diéthylamino)éthoxy]éthanol ou un sel de ce dérivé.

**9.** Procédé de fabrication d'un dérivé de 1,2-éthanediol représenté par la formule générale suivante, ou d'un sel de ce dérivé :

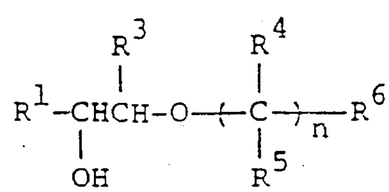

formule dans laquelle :
- $R^1$ représente un groupe hétérocyclique substitué ou non substitué ;
- $R^3$ représente un atome d'hydrogène ou un groupe alkyle en $C_{1-6}$ ;
- les $nR^4$ et les $nR^5$ sont identiques ou différents les uns des autres et représentent des atomes d'hydrogène ou des groupes alkyle en $C_{1-6}$ ;
- $R^6$ représente un groupe ammonio ou un groupe amino ou hétérocyclique contenant de l'azote, substitué ou non substitué, le groupe hétérocyclique contenant de l'azote étant choisi dans le groupe constitué par les groupes pyrrolyle, pyrrolidinyle, pipéridyle, pipérazinyle, imidazolyle, pyrazolyle, pyridyle, tétrahydropyridyle, pyrimidinyle, morpholinyle, thiomorpholinyle, quinolyle, quinolizinyle, tétrahydroquinolinyle, tétrahydroisoquinolinyle, quinuclidinyle, thiazolyle, tétrazolyle, thiadiazolyle, pyrrolinyle, imidazolinyle, imidazolidinyle, pyrazolinyle, pyrazolidinyle, purinyle et indazolyle ; et
- n représente 0 ou un nombre entier de 1 à 6,

le substituant sur $R^1$ étant choisi dans le groupe constitué par les atomes d'halogène, les groupes amino, alkyle en $C_{1-6}$, aryle, ar-alkyle en $C_{1-4}$, alcoxy en $C_{1-6}$, ar-alcoxy en $C_{1-4}$, aryloxy, carbamoyloxy, alkylthio en $C_{1-6}$, alcényle en $C_{2-6}$, alcényloxy en $C_{2-6}$, ar-alkylthio en $C_{1-4}$, ar-alkylsulfonyle en $C_{1-6}$, arylsulfonyle, alkylsulfonylamino en $C_{1-6}$, arylsulfonylamino et hétérocycliques, substitués ou non substitués, les groupes amino protégés, les groupes hydroxyle protégés ou non

protégés, le groupe nitro, le groupe oxo et les groupes alkylènedioxy en $C_{1-4}$ ;
le groupe alkyle en $C_{1-6}$, aryle, ar-alkyle en $C_{1-4}$, alcoxy en $C_{1-6}$, ar-alcoxy en $C_{1-4}$, aryloxy, carbamoyloxy, alkythio en $C_{1-6}$, alcényle en $C_{2-6}$, alcényloxy en $C_{2-6}$, ar-alkylthio en $C_{1-4}$, ar-alkylsulfonyle en $C_{1-6}$, arylsulfonyle, alkylsulfonylamino en $C_{1-6}$, arylsulfonylamino ou hétérocyclique, substitué, en tant que substituant de $R^1$, et le groupe hétérocyclique contenant de l'azote, substitué, en tant que $R^6$, ayant chacun au moins un substituant choisi dans le groupe constitué par les atomes d'halogène, les groupes hydroxyle protégés ou non protégés, les groupes amino protégés ou non protégés, les groupes carboxyle protégés ou non protégés, les groupes alkyle en $C_{1-6}$ non substitués, les groupes alkyle en $C_{1-6}$ substitués par un groupe hydroxyle protégé ou non protégé, les groupes aryle non substitués ou substitués par halogène, les groupes aroyle non substitués ou substitués par halogène, les groupes alcoxy en $C_{1-6}$ non substitués, les groupes alcoxy en $C_{1-6}$ substitués par un groupe alcoxy en $C_{1-6}$, les groupes acyle en $C_{1-6}$ les groupes ar-alkyle en $C_{1-4}$, les groupes aralcényle en $C_{2-6}$, les groupes hétérocycliques, les groupes hétérocyclique-CO-, le groupe oxo, les groupes alkylsulfonyle en $C_{1-6}$ et les groupes arylsulfonyle ; et
le groupe amino substitué, en tant que substituant de $R^1$, et le groupe amino substitué, en tant que $R^6$, ayant chacun au moins un substituant choisi dans le groupe constitué par les groupes hydroxyle protégés ou non protégés, les groupes alkyle en $C_{1-6}$ non substitués, les groupes alkyle en $C_{1-6}$ substitués par un groupe carboxyle ou hydroxyle, protégé ou non protégé, les groupes cycloalkyle, les groupes aryle, les groupes acyle en $C_{1-6}$, les groupes ar-alkyle en $C_{1-4}$, les groupes hétérocycliques, les groupes hétérocyclique-CO- non substitués ou substitués par oxo, le groupe adamantyle, les groupes alkylsulfonyle en $C_{1-6}$ et les groupes arylsulfonyle; tous les groupes hétérocycliques ci-dessus étant choisis dans le groupe constitué par les groupes hétérocycliques contenant de l'azote mentionnés dans la définition de $R^6$ et les groupes furyle, thiényle, benzothiényle, pyrannyle, isobenzofurannyle, oxazolyle, benzofurannyle, indolyle, benzimidazolyle, benzoxazolyle, benzothiazolyle, quinoxalyle, dihydroquinoxalyle, 2,3-dihydrobenzothiényle, 2,3-dihydrobenzopyrrolyle, 2,3-dihydro-4H-1-thianaphtyle, 2,3-dihydrobenzofurannyle, benzo[b]dioxannyle, imidazo [2,3-a]pyridyle, benzo[b]pipérazinyle, chroményle, isothiazolyle isoxazolyle, oxadiazolyle, pyridazinyle, isoindolyle et isoquinolyle,
qui comprend la réaction d'un composé représenté par la formule générale :

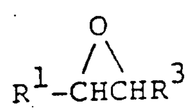

dans laquelle $R^1$ et $R^3$ ont la même signification que celle définie ci-dessus,
avec un composé représenté par la formule générale suivante ou un sel de ce composé :

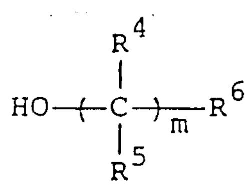

dans laquelle :

- les $mR^4$ et les $mR^5$ sont identiques ou différents les uns des autres et représentent des atomes d'hydrogène ou des groupes alkyle inférieurs ;
- $R^6$ a la même signification que celle définie ci-dessus; et
- m représente un nombre entier de 1 à 6;

ou un composé représenté par la formule générale suivante ou un sel de ce composé :

$HO\text{-}R^{6a}$

dans laquelle $R^{6a}$ représente le même groupe hétérocyclique contenant de l'azote, substltué ou non substitué, que dans la définition de $R^6$, à la condition que le groupe hétérocyclique présente une

valence libre sur un atome de carbone formant le noyau hétérocyclique.

**10.** Procédé de fabrication d'un dérivé de 1,2-éthanediol, représenté par la formule générale suivante, ou d'un sel de ce dérivé :

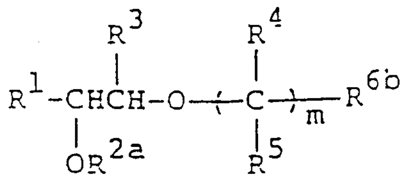

dans laquelle :

- $R^1$ représente un groupe hétérocyclique substitué ou non substitué ;
- $R^{2a}$ représente un groupe protecteur d'hydroxyle ;
- $R^3$ représente un atome d'hydrogène ou un groupe alkyle en $C_{1-6}$ ;
- les $mR^4$ et les $mR^5$ sont identiques ou différents les uns des autres et représentent des atomes d'hydrogène ou des groupes alkyle en $C_{1-6}$ ;
- $R^{6b}$ représente un groupe amino ou hétérocyclique contenant de l'azote, substitué ou non substitué, le groupe hétérocyclique contenant de l'azote ayant une valence libre sur un atome d'azote formant le noyau hétérocyclique et étant choisi dans le groupe constitué par les groupes pyrrolyle, pyrrolidinyle, pipéridyle, pipérazinyle, imidazolyle, pyrazolyle, pyridyle, tétrahydropyridyle, pyrimidinyle, morpholinyle, thiomorpholinyle, quinolyle, quinolizinyle, tétrahydroquinolinyle, tétrahydroisoquinolinyle, quinuclidinyle, thiazolyle, tétrazolyle, thiadiazolyle, pyrrolinyle, imidazolinyle, imidazolidinyle, pyrazolinyle, pyrazolidinyle, purinyle et indazolyle ; et
- m représente un nombre entier de 1 à 6,

le substituant sur $R^1$ étant choisi dans le groupe constitué par les atomes d'halogène, les groupes amino, alkyle en $C_{1-6}$, aryle, ar-alkyle en $C_{1-4}$, alcoxy en $C_{1-6}$, ar-alcoxy en $C_{1-4}$, aryloxy, carbamoyloxy, alkylthio en $C_{1-6}$, alcényle en $C_{2-6}$, alcényloxy en $C_{2-6}$, ar-alkylthio en $C_{1-4}$, ar-alkylsulfonyle en $C_{1-6}$, arylsulfonyle, alkylsulfonylamino en $C_{1-6}$, arylsulfonylamino et hétérocycliques, substitués ou non substitués, les groupes amino protégés, les groupes hydroxyle protégés ou non protégés, le groupe nitro, le groupe oxo et les groupes alkylènedioxy en $C_{1-4}$ ;
le groupe alkyle en $C_{1-6}$, aryle, ar-alkyle en $C_{1-4}$, alcoxy en $C_{1-6}$, ar-alcoxy en $C_{1-4}$, aryloxy, carbamoyloxy, alkylthio en $C_{1-6}$, alcényle en $C_{2-6}$, alcényloxy en $C_{2-6}$, ar-alkylthio en $C_{1-4}$, ar-alkylsulfonyle en $C_{1-6}$, arylsulfonyle, alkylsulfonylamino en $C_{1-6}$, arylsulfonylamino ou hétérocyclique, substitué, en tant que substituant de $R^1$, et le groupe hétérocyclique contenant de l'azote, substitué, en tant que $R^{6b}$, ayant chacun au moins un substituant choisi dans le groupe constitué par les atomes d'halogène, les groupes hydroxyle protégés ou non protégés, les groupes amino protégés ou non protégés, les groupes carboxyle protégés ou non protégés, les groupes alkyle en $C_{1-6}$ non substitués, les groupes alkyle en $C_{1-6}$ substitués par un groupe hydroxyle protégé ou non protégé, les groupes aryle non substitués ou substitués par halogène, les groupes aroyle non substitués ou substitués par halogène, les groupes alcoxy en $C_{1-6}$ non substitués, les groupes alcoxy en $C_{1-6}$ substitués par un alcoxy en $C_{1-6}$, les groupes acyle en $C_{1-6}$, les groupes aralkyle en $C_{1-4}$, les groupes ar-alcényle en $C_{2-6}$, les groupes hétérocycliques, les groupes hétérocyclique-CO-, le groupe oxo, les groupes alkylsulfonyle en $C_{1-6}$ et les groupes arylsulfonyle ; et
le groupe amino substitué, en tant que substituant de $R^1$, et le groupe amino substitué, en tant que $R^{6b}$, ayant chacun au moins un substituant choisi dans le groupe constitué par les groupes hydroxyle protégés ou non protégés, les groupes alkyle en $C_{1-6}$ non substitués, les groupes alkyle en $C_{1-6}$ substitués par un groupe carboxyle ou hydroxyle, protégé ou non protégé, les groupes cycloalkyle, les groupes aryle, les groupes acyle en $C_{1-6}$, les groupes ar-alkyle en $C_{1-4}$, les groupes hétérocycliques, les groupes hétérocyclique-CO- non substitués ou substitués par oxo, le groupe adamantyle, les groupes alkylsulfonyle en $C_{1-6}$ et les groupes arylsulfonyle; tous les groupes hétérocycliques ci-dessus étant choisis dans le groupe constitué par les groupes hétérocycliques contenant de l'azote mentionnés dans la définition de $R^{6b}$ et les groupes furyle, thiényle, benzothiényle, pyrannyle, isobenzofurannyle, oxazolyle, benzofurannyle, indolyle, benzimidazolyle, benzoxazolyle, benzothiazolyle, quinoxalyle, dihydroquinoxalyle, 2,3-dihydrobenzothiényle, 2,3-dihydrobenzopyrrolyle, 2,3-dihydro-4H-1-thianaphtyle, 2,3-dihydrobenzofurannyle, benzo[b]dioxannyle, imidazo [2,3-a]pyridyle, benzo[b]-pipérazinyle, chroményle, isothiazolyle, isoxazolyle, oxadiazolyle, pyridazinyle, isoindolyle et isoquinolyle,
qui comprend la réaction d'un composé représenté par la formule générale :

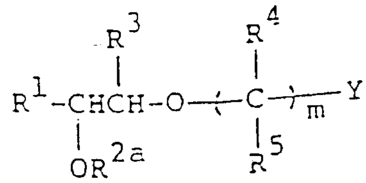

dans laquelle :

- $R^1$, $R^{2a}$, $R^3$, $R^4$, $R^5$ et m ont les mêmes significations que celles définies ci-dessus ; et
- Y représente un groupe éliminable,

avec un composé représenté par la formule générale suivante, ou un sel de ce composé :

$H\text{-}R^{6b}$

dans laquelle $R^{6b}$ a la même signification que celle définie ci-dessus.

**11.** Utilisation d'un dérivé de 1,2-éthanediol représenté par la formule générale suivante, ou d'un sel de ce dérivé, pour la préparation d'un médicament pour le traitement de la démence cérébro-vasculaire, de la démence sénile, de la démence d'Alzheimer, des séquelles de l'encéphalopathie ischémique ou de l'apoplexie cérébrale chez un patient :

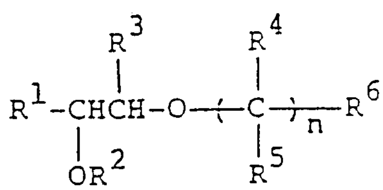

formule dans laquelle $R^1$, $R^2$, $R^3$, $R^4$, $R^5$, $R^6$ et n sont tels que définis à la revendication 1.

**12.** Utilisation d'un dérivé de 1,2-éthanediol, ou d'un sel de ce dérivé, selon la revendication 11, dans laquelle :

- $R^1$ représente un groupe furyle, thiényle, pyridyle, quinolyle, benzothiényle, 2,3-dihydrobenzofurannyle, indolyle, benzofurannyle, 2,3-dihydrobenzothiényle, 1,2,3,4-tétrahydroquinolinyle ou imidazolyle, substitué ou non substitué ;
- $R^6$ représente un groupe ammonio ou un groupe amino ou hétérocyclique contenant de l'azote, substitué ou non substitué, le groupe hétérocyclique contenant de l'azote étant choisi dans le groupe constitué par pyrrolinyle, pipéridyle, pipérazinyle, imidazolyle, pyridyle, morpholinyle, thiomorpholinyle, tétrahydropyridyle, quinuclidinyle et tétrazolyle ;
- le substituant sur $R^1$ est choisi dans le groupe constitué par les atomes d'halogène ; les groupes alkyle en $C_{1-6}$ ; les groupes alcényle en $C_{2-6}$ ; les groupes phényle qui peuvent être facultativement substitués par un atome d'halogène ou un groupe alkyle en $C_{1-6}$ ou alcoxy en $C_{1-6}$ ; les groupes phényl-alkyle en $C_{1-4}$ ; les groupes alcoxy en $C_{1-6}$ qui peuvent facultativement être substitués par un groupe pyridyle, furyle ou thiényle ; les groupes phényl-alcoxy en $C_{1-4}$ qui peuvent facultativement être substitués par un atome d'halogène ou un groupe alkyle en $C_{1-6}$, hydroxyle ou alcoxy en $C_{1-6}$ ; un groupe phénoxy ; les groupes phénylaminocarbonyloxy qui peuvent facultativement être substitués par un atome d'halogène; les groupes alkyle en $C_{1-6}$-aminocarbonyloxy; les groupes alkylthio en $C_{1-6}$; un groupe phénylsulfonylamino ; les groupes amino qui peuvent facultativement être substitués par un groupe alkyle en $C_{1-6}$ ; un groupe hydroxyle ; un groupe nitro; un groupe oxo ; les groupes phényl-alkylthio en $C_{1-4}$ ; les groupes phényl-alkylsulfonyle en $C_{1-4}$ ; un groupe thiényle ; un groupe pyridyle ; et les groupes alkylènedioxy en $C_{1-4}$ ;

- le substituant sur $R^6$, lorsque $R^6$ est un groupe amino substitué, est choisi dans le groupe constitué par les groupes alkyle en $C_{1-6}$ qui peuvent facultativement être substitués par un groupe hydroxyle ; les groupes acyle en $C_{1-6}$ ; les groupes cycloalkyle ; les groupes phénylalkyle en $C_{1-4}$ ; un groupe pyrimidinyle ; un groupe pyridinecarbonyle et un groupe adamantyle ; et
- le substituant sur $R^6$, lorsque $R^6$ est un groupe hétérocyclique contenant de l'azote, substitué, est choisi dans le groupe constitué par les atomes d'halogène ; un groupe hydroxyle ; les groupes alkyle en $C_{1-6}$ qui peuvent facultativement être substitués par un groupe hydroxyle ; un groupe amino ; les groupes acyle en $C_{1-6}$ qui peuvent facultativement être substitués par un groupe pyrrolidinyle ; les groupes phényl-alkyle en $C_{1-4}$ ; les groupes phényl-alcényle en $C_{2-4}$ ; les groupes aroyle qui sont substitués par un atome d'halogène ; un groupe pyridyle et un groupe oxo.

**13.** Utilisation d'un dérivé de 1,2-éthanediol, ou d'un sel de ce dérivé, selon la revendication 12, dans laquelle :
- $R^1$ représente un groupe benzothiényle, benzofurannyle ou 2,3-dihydrobenzothiényle ;
- $R^2$ représente un atome d'hydrogène ;
- $R^3$ représente un atome d'hydrogène ;
- les $nR^4$ et $nR^5$ représentent des atomes d'hydrogène ;
- $R^6$ représente un groupe pyridyle ; un groupe pipéridinyle qui peut facultativement être substitué par un groupe alkyle en $C_{1-6}$ ; un groupe 1,2,5,6-tétrahydropyridyle qui peut facultativement être substitué par un groupe alkyle en $C_{1-6}$ ; un groupe imidazolyle ; un groupe pipérazinyle substitué par un groupe phényl-alkyle en $C_{1-4}$ ou un groupe amino qui peut facultativement être substitué par un groupe alkyle en $C_{1-6}$ ou un groupe cycloalkyle ; et
- n représente un nombre entier de 1 à 4.

**14.** Utilisation d'un dérivé de 1,2-éthanediol, ou d'un sel de ce dérivé, selon la revendication 11, dans laquelle le dérivé ou sel est le 1-(benzo[b]thiophène-5-yl)-2-[2-(N,N-diméthylamino)éthoxy]éthanol ou un sel de ce dérivé.

**15.** Utilisation d'un dérivé de 1,2-éthanediol, ou d'un sel de ce dérivé, selon la revendication 11, dans laquelle le dérivé ou sel est le 1-(benzo[b]thiophène-5-yl)-2-[3-(N,N-diméthylamino)propoxy]éthanol ou un sel de ce dérivé.

**16.** Utilisation d'un dérivé de 1,2-éthanediol, ou d'un sel de ce dérivé, selon la revendication 11, dans laquelle le dérivé ou sel est le 1-(benzo[b]thiophène-5-yl)-2-(imidazolylméthoxy)éthanol ou un sel de ce dérivé.

**17.** Utilisation d'un dérivé de 1,2-éthanediol, ou d'un sel de ce dérivé, selon la revendication 11, dans laquelle le dérivé ou sel est le 1-(benzo[b]thiophène-5-yl)-2-[(1-méthyl-1,2,5,6-tétrahydropyridine-3-yl)-méthoxy]éthanol ou un sel de ce dérivé.

**18.** Utilisation d'un dérivé de 1,2-éthanediol, ou d'un sel de ce dérivé, selon la revendication 11, dans laquelle le dérivé ou sel est le 1-(benzo[b]furanne-5-yl)-2-[2-(N,N-diméthylamino)éthoxy]éthanol ou un sel de ce dérivé.

**19.** Utilisation d'un dérivé de 1,2-éthanediol, ou d'un sel de ce dérivé, selon la revendication 11, dans laquelle le dérivé ou sel est le 1-(benzo[b]thiophène-5-yl)-2-[2-(N,N-diéthylamino)-éthoxy]éthanol ou un sel de ce dérivé.

**20.** Agent améliorant la fonction cérébrale comprenant un dérivé de 1,2-éthanediol représenté par la formule générale suivante, ou un sel de ce dérivé :

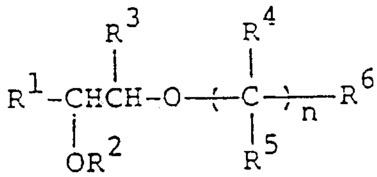

dans laquelle $R^1$, $R^2$, $R^3$, $R^4$, $R^5$, $R^6$ et n sont tels que définis à la revendication 1.